(19) Europäisches Patentamt
European Patent Office
Office européen des brevets

(11) EP 1 038 873 B1

(12) **FASCICULE DE BREVET EUROPEEN**

(45) Date de publication et mention de la délivrance du brevet: **02.05.2002 Bulletin 2002/18**

(51) Int Cl.[7]: **C07D 405/12**, A61K 31/40, A61P 25/00, C07D 403/12, C07D 209/70, C07D 409/12

(21) Numéro de dépôt: **00400812.4**

(22) Date de dépôt: **24.03.2000**

(54) **Dérivés du benzo[3,4]cyclobuta[1,2-c]pyrrole en tant qu' inhibiteurs de recapture de sérotonine**

Derivate von Benzo(1,2-c)pyrrol als Inhibitoren der Serotonin Wideraufnahme

Benzo(3,4)cyclobuta(1,2-c)pyrrole derivatives as inhibitors of serotonine reuptake

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **26.03.1999 FR 9903811**

(43) Date de publication de la demande:
**27.09.2000 Bulletin 2000/39**

(73) Titulaire: **LES LABORATOIRES SERVIER**
**92200 Neuilly sur Seine (FR)**

(72) Inventeurs:
- **Peglion, Jean-Louis**
  **78110 Le Vesinet (FR)**
- **Goument, Bertrand**
  **78220 Viroflay (FR)**
- **Dessinges, Aimée**
  **92500 Rueil-Malmaison (FR)**
- **Millan, Mark**
  **78230 Le Pecq (FR)**
- **Rivet, Jean-Michel**
  **92000 Nanterre (FR)**
- **Dekeyne, Anne**
  **78470 Saint Remy Les Chevreuses (FR)**

(56) Documents cités:
**EP-A- 0 790 250**
**US-A- 4 314 081**
**US-A- 4 721 723**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 038 873 B1

## Description

La présente invention concerne de nouveaux dérivés du benzo[3,4]cyclobuta[1,2-c]pyrrole, leur procédé de préparation et les compositions pharmaceutiques qui les contiennent.
Les composés de la présente invention agissent comme de puissants inhibiteurs de recapture de sérotonine.
A ce titre ils peuvent être utilisés en thérapeutique pour le traitement de la dépression, des attaques de panique, des troubles obsessionnels compulsifs, des phobies, des troubles impulsifs, de l'abus de drogue et de l'anxiété.
En effet, les résultats des expériences de microdialyse réalisées dans le cortex frontal montrent l'intérêt des produits de la présente invention dans les traitements des différentes pathologies du système nerveux central. Entraînant dans ce territoire une augmentation de la libération de sérotonine, ils sont tout à fait aptes à être utilisés dans des pathologies qui sont liées à un défaut de transmission de ce neuromédiateur.
D'autre part les tests réalisés sur des souris agressives permettent de mettre en évidence les propriétés anti-impulsives et anxiolytiques des produits de l'invention.
Des inhibiteurs de recapture de monoamines telles que la sérotonine ont déjà été décrits dans US-A-4 314 081. Le brevet US-A-4 721 723 décrit des sels de parotexine également inhibiteurs de la recapture de sérotonine.
Plus particulièrement, la présente invention concerne les composés de formule (I), à jonction de cycle cis :

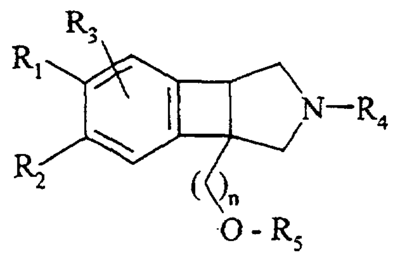

**(I)**

dans laquelle :

**$R_1$, $R_2$, $R_3$,** identiques ou différents, indépendamment l'un de l'autre, représentent un groupement choisi parmi atome d'hydrogène, d'halogène, groupement alkyle ($C_1$-$C_6$) linéaire ou ramifié, alkényle ($C_2$-$C_6$) linéaire ou ramifié, alkynyl ($C_2$-$C_6$) linéaire ou ramifié, hydroxy, alkoxy ($C_1$-$C_6$) linéaire ou ramifié, cycloalkyle, cycloalkylalkyle ($C_1$-$C_6$) linéaire ou ramifié, aryle, arylalkyle ($C_1$-$C_6$) linéaire ou ramifié, aryloxy, arylalkoxy ($C_1$-$C_6$) linéaire ou ramifié, trihalogénoalkyle ($C_1$-$C_6$) linéaire ou ramifié, trihalogénoalkoxy ($C_1$-$C_6$) linéaire ou ramifié, cyano, nitro, un groupement de formule - $OSO_2CH_3$, -$OSO_2CF_3$, $NR_6R_7$, $CO_2R_6$ dans lesquels $R_6$ et $R_7$, identiques ou différents. indépendamment l'un de l'autre, représentent un atome d'hydrogène, un groupement alkyle ($C_1$-$C_6$) linéaire ou ramifié, alkényle ($C_2$-$C_6$) linéaire ou ramifié, alkynyle ($C_2$-$C_6$) linéaire ou ramifié, cycloalkyle, cycloalkylalkyl ($C_1$-$C_6$) linéaire ou ramifié, aryle, arylalkyle ($C_1$-$C_6$) linéaire ou ramifié, ou $R_1$, $R_2$, $R_3$ pris par deux en positions adjacentes, représentent, avec les atomes communs du cycle benzénique auquel ils sont liés, un système cyclique ($C_4$-$C_8$), saturé ou insaturé, dont éventuellement un ou deux atomes de carbone est (sont) remplacé(s) par un ou deux hétéroatomes, identiques ou différents, choisis parmi oxygène, azote et soufre,
étant entendu que dans le cas où $R_1$, $R_2$, $R_3$ sont pris par deux en positions adjacentes et représentent la définition précédemment citée, alors le groupe restant $R_1$ ou $R_2$ ou $R_3$ prend l'une des définitions individuelles de ces groupements précédemment mentionnées,

**$R_4$** représente un groupement choisi parmi atome d'hydrogène, groupement alkyle ($C_1$-$C_6$) linéaire ou ramifié, alkényle ($C_2$-$C_6$) linéaire ou ramifié, alkynyle ($C_2$-$C_6$) linéaire ou ramifié, cycloalkyle, cycloalkylalkyle ($C_1$-$C_6$) linéaire ou ramifié, aryle, arylalkyle ($C_1$-$C_6$) linéaire ou ramifié, hétérocycloalkyle, hétérocycloalkylalkyle ($C_1$-$C_6$) linéaire ou ramifié, hétéroaryle et hétéroarylalkyle ($C_1$-$C_6$) linéaire ou ramifié,

**$R_5$** représente un groupement aryle, aryle éventuellement substitué, hétéroaryle ou hétéroaryle éventuellement substitué,

**n** représente un entier compris entre 1 et 3 inclus,

leurs isomères, ainsi que leurs sels d'addition à un acide ou à une base pharmaceutiquement acceptable,
étant entendu que par groupement cycloalkyle, on comprend un système cyclique ($C_3$-$C_8$), mono ou bicyclique, com-

portant éventuellement une ou plusieurs insaturation(s), ladite ou lesdites insaturations ne conférant pas un caractère aromatique au système cyclique, éventuellement substitué par un ou plusieurs groupements, identiques ou différents, choisis parmi atome d'halogène, et groupement alkyle ($C_1$-$C_6$) linéaire ou ramifié,

étant entendu que par groupement hétérocycloalkyle, on comprend un groupement cycloalkyle éventuellement substitué tel que défini précédemment et contenant au niveau du système cyclique un, deux ou trois hétéroatomes, identiques ou différents, choisis parmi oxygène, azote et soufre,

étant entendu aussi que par groupement aryle, on comprend un groupement phényle, naphtyle, dihydronaphtyle, tétrahydronaphtyle, indényle ou indanyle, et que par groupement aryle éventuellement substitué, on comprend un groupement aryle tel que défini éventuellement substitué par un ou plusieurs groupements, identiques ou différents, choisis parmi halogène, hydroxy, alkyle ($C_1$-$C_6$) linéaire ou ramifié, alkoxy ($C_1$-$C_6$) linéaire ou ramifié, alkényle ($C_1$-$C_6$) linéaire ou ramifié, cycloalkyle, adamantyle, cycloalkylalkyle ($C_1$-$C_6$) linéaire ou ramifié, aryle, arylalkyl ($C_1$-$C_6$) linéaire ou ramifié, aryloxy, arylalkoxy ($C_1$-$C_6$) linéaire ou ramifié, trihalogénoalkyle ($C_1$-$C_6$) linéaire ou ramifié, trihalogénoalkoxy ($C_1$-$C_6$) linéaire ou ramifié, cyano, nitro, oxo, un groupement de formule $-OSO_2CH_3$, $-OSO_2CF_3$, $NR_6R_7$, ou $CO_2R_6$ dans lesquels $R_6$ et $R_7$ sont tels que définis dans la formule (I),

étant entendu également que par groupement hétéroaryle, on comprend un groupement aryle tel que défini précédemment, contenant un, deux ou trois hétéroatomes, identiques ou différents, choisis parmi oxygène, azote et soufre, et que par groupement hétéroaryle éventuellement substitué, on comprend un groupement hétéroaryle tel que défini précédemment éventuellement substitué par un ou plusieurs groupements, identiques ou différents, tels que définis pour les substitutions des groupements aryles.

Parmi les acides pharmaceutiquement acceptables, on peut citer à titre non limitatif les acides chlorhydrique, bromhydrique, sulfurique, phosphonique, acétique, trifluoroacétique. lactique, pyruvique, malonique, glutarique, succinique, fumarique, tartrique, maléïque. citrique, ascorbique, oxalique, méthane sulfonique, camphorique, etc...

Parmi les bases pharmaceutiquement acceptables, on peut citer, à titre non limitatif, l'hydroxyde de sodium, l'hydroxyde de potassium, la triéthylamine, la tertbutylamine, etc...

Les substituants $R_5$ préférés, selon l'invention sont les groupements hétéroaryles. Selon une variante avantageuse, les groupements $R_5$ préférés sont les groupements méthylènedioxyphényle et éthylènedioxyphényle.

Selon une variante avantageuse de l'invention, n représente préférentiellement un entier égal à 1. Selon une autre variante avantageuse de l'invention, n représente préférentiellement un entier égal à 2.

Les substituants $R_4$ préférés, selon l'invention, sont l'atome d'hydrogène, les groupements alkyle ($C_1$-$C_6$) linéaire ou ramifié, et arylalkyle ($C_1$-$C_6$) linéaire ou ramifié.

Les composés préférés selon l'invention sont :

- le *cis*-3a-[(3,4-méthylènedioxyphényloxy)-méthyl]-2,3,3a,7b-tétrahydro-1*H*-benzo[3,4] cyclobuta[1,2-c]pyrrole,
- le *cis*-2-benzyl-5-méthoxy-3a-[(3,4-méthylènedioxyphényloxy)-méthyl]-2,3,3a,7b-tétrahydro-1*H*-benzo[3,4]cyclobuta[1,2-c]pyrrole,
- le *cis*-5-méthoxy-3a-[(3,4-méthylènedioxyphényloxy)-méthyl]-2,3,3a,7b-tétrahydro-1*H*-benzo[3,4]cyclobuta [1,2-c]pyrrole,
- le *cis*-5-méthoxy-2-méthyl-3a-[(3,4-méthylènedioxyphényloxy)-méthyl]-2,3,3a,7b-tétrahydro-1*H*-benzo[3,4]cyclobuta[1,2-c]pyrrole,
- le *cis*-6-trifluorométhyl-3a-[(3,4-méthylènedioxyphényloxy)-méthyl]-2,3,3a,7b-tétra hydro-1*H*-benzo[3,4]cyclobuta [1,2-c]pyrrole,
- et le *cis*-6-fluoro-3a-[(3,4-méthylènedioxyphényloxy)-méthyl]-2,3,3a,7b-tétrahydro-1*H*-benzo[3,4]cyclobuta[1,2-c] pyrrole.

Les isomères ainsi que les sels d'additions à un acide ou à une base pharmaceutiquement acceptable des composés préférés font partie intégrante de l'invention.

L'invention s'étend également au procédé de préparation des composés de formule **(I),** caractérisé en ce qu'on utilise comme produit de départ un composé de formule **(II) :**

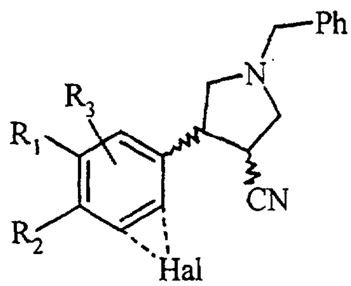

(II)

dans laquelle $R_1$, $R_2$, $R_3$ ont la même signification que dans la formule **(I)** et Hal représente un atome d'halogène, ledit atome d'halogène étant fixé sur l'un ou l'autre atome de carbone du cycle benzénique, composés de formule **(II)** que l'on soumet à l'action de l'amidure de sodium dans l'ammoniaque liquide, pour conduire aux composés de formule **(III),** à jonction de cycle *cis,*

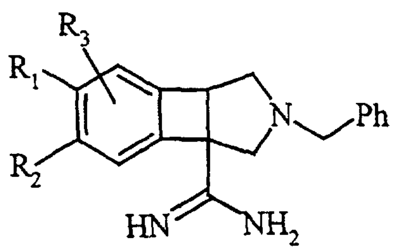

(III)

dans laquelle $R_1$, $R_2$, $R_3$ ont la même signification que dans la formule **(I),**
composés de formule **(III)** que l'on traite par une base minérale, en milieu alcoolique aqueux, pour conduire aux composés de formule **(IV),** à jonction de cycle *cis,*

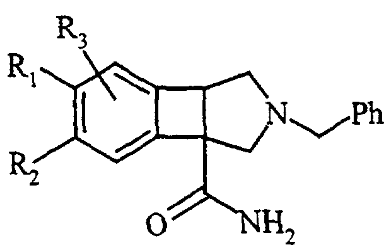

(IV)

dans laquelle $R_1$, $R_2$, $R_3$ ont la même signification que dans la formule **(I),**
composés de formule **(IV)** que l'on transforme par l'intermédiaire d'un acétal de N,N-diméthylformamide de formule **(V)**

$$(CH_3)_2N\ CH(OG)_2 \qquad \textbf{(V)}$$

dans laquelle G représente un groupement alkyle ($C_1$-$C_6$) linéaire ou ramifié, benzyle ou cyclohexyle,
en ester de formule **(VI),** à jonction de cycle *cis,*

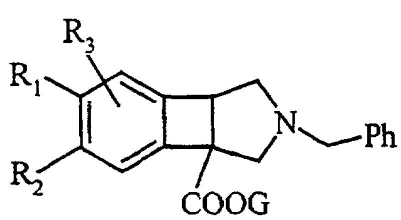

(VI)

dans laquelle $R_1$, $R_2$, $R_3$, et G sont tels que définis précédemment, composés de formule **(VI)** qui sont réduits, selon des conditions classiques de synthèse organique, en alcool de formule **(VII),** à jonction de cycle *cis*,

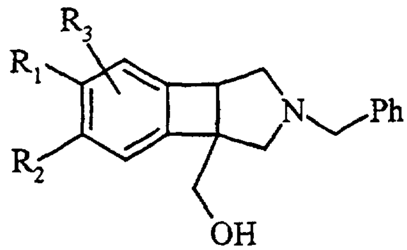

**(VII)**

dans laquelle $R_1$, $R_2$, $R_3$ sont tels que définis précédemment, composés de formule **(VII)** qui sont :

→ soit traités selon les conditions de la réaction de Mitsunobu par un composé de formule **(VIII)**

$R_5$ - OH **(VIII)**

dans laquelle $R_5$ a la même signification que dans la formule **(I)** pour conduire aux composés de formule **(I/a),** à jonction de cycle *cis,* cas particulier des composés de formule (I) :

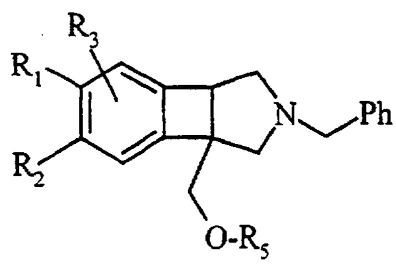

**(I/a)**

dans laquelle $R_1$, $R_2$, $R_3$ et $R_5$ sont tels que définis précédemment, composés de formule **(I/a)** qui peuvent être débenzylés par une des méthodes classiquement utilisées en synthèse organique, pour conduire aux composés de formule **(I/b),** à jonction de cycle *cis,* cas particulier des composés de formule (I) :

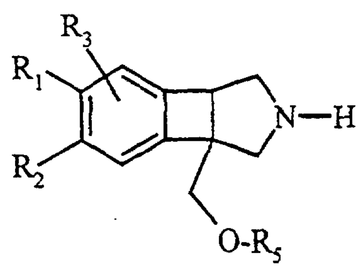

**(I/b)**

dans laquelle $R_1$, $R_2$, $R_3$ et $R_5$ sont tels que définis précédemment, composés de formule **(I/b)** qui peuvent être :

* soit transformés par traitement par le formaldéhyde en présence de cyanoborohydrure de sodium en composés de formule **(I/c),** à jonction de cycle *cis,* cas particulier des composés de formule (I) :

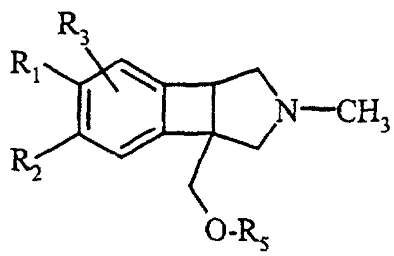

**(I/c)**

dans laquelle $R_1$, $R_2$, $R_3$ et $R_5$ sont tels que définis précédemment,

* soit mis en présence, selon des conditions classiques de synthèse organique, d'un composé de formule (**IX**) :

$R'_4$ - Z **(IX)**

dans lequel $R'_4$ a les mêmes définitions que $R_4$ à l'exception de hydrogène et benzyle, et Z représente un groupement partant usuellement utilisé en synthèse organique,
pour conduire aux composés de formule **(I/d),** à jonction de cycle *cis*, cas particulier des composés de formule (I) :

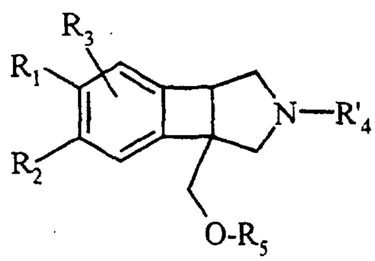

**(I/d)**

dans laquelle $R_1$, $R_2$, $R_3$, $R'_4$ et $R_5$ sont tels que définis précédemment,

→ soit transformés, par une suite de réactions classiques, en composés de formule **(X),** à jonction de cycle *cis*,

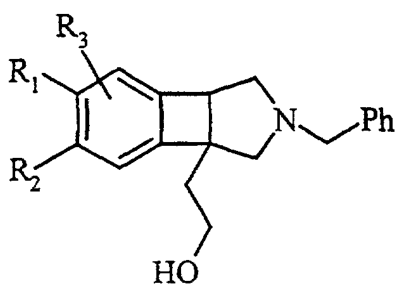

**(X)**

dans laquelle $R_1$, $R_2$, $R_3$ sont tels que définis précédemment,
composés de formule **(X)** qui sont traités selon les conditions de la réaction de Mitsunobu par un composé de formule **(VIII) :**

$R_5$ - OH **(VIII)**

dans laquelle $R_5$ est tel que défini précédemment,
pour conduire aux composés de formule (**I/e**), à jonction de cycle *cis*, cas particulier des composés de formule (I) :

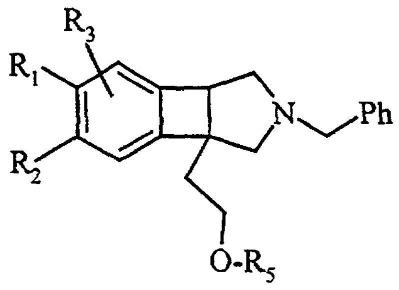

**(I/e)**

dans laquelle $R_1$, $R_2$, $R_3$ et $R_5$ sont tels que définis précédemment, composés de formule **(I/e)** qui peuvent être débenzylés par une des méthodes classiquement utilisées en synthèse organique, pour conduire aux composés de formule **(I/f),** à jonction de cycle *cis,* cas particulier des composés de formule (I) :

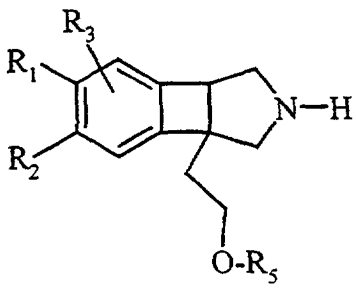

**(I/f)**

dans laquelle $R_1$, $R_2$, $R_3$ et $R_5$ sont tels que définis précédemment, composés de formule **(I/f)** qui peuvent être :

* soit transformés par traitement par le formaldéhyde en présence de cyanoborohydrure de sodium en composés de formule **(I/g),** à jonction de cycle *cis*, cas particulier des composés de formule (I) :

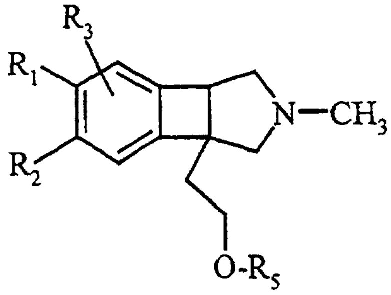

**(I/g)**

dans laquelle $R_1$, $R_2$, $R_3$ et $R_5$ sont tels que définis précédemment,

* soit mis en présence, selon des conditions classiques de synthèse organique, d'un composé de formule **(IX) :**

$R'_4$ - Z **(IX)**

dans lequel $R'_4$ a les mêmes définitions que $R_4$ à l'exception de hydrogène et benzyle, et Z représente un groupement partant usuellement utilisé en synthèse organique, pour conduire aux composés de formule **(I/h),** à jonction de cycle *cis,* cas particulier des composés de formule (I) :

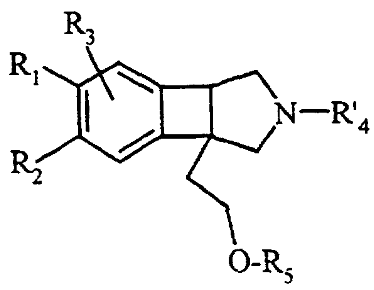

**(I/h)**

dans laquelle $R_1$, $R_2$, $R_3$, $R'_4$ et $R_5$ sont tels que définis précédemment,

→ soit transformés par une suite de réactions classiques en composés de formule **(XI),** à jonction de cycle *cis*, :

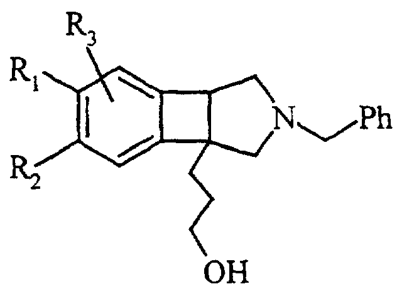

**(XI)**

dans laquelle $R_1$, $R_2$, $R_3$, sont tels que définis précédemment,
les composés de formule (XI) pouvant également être obtenus par une suite de réactions classiques à partir des composés de formule (X),
composés de formule **(XI)** qui sont traités selon les conditions de la réaction de Mitsunobu par un composé de formule **(VIII) :**

$$R_5 - OH \qquad \textbf{(VIII)}$$

dans laquelle $R_5$ est tel que défini précédemment,
pour conduire aux composés de formule **(I/i),** à jonction de cycle *cis,* cas particulier des composés de formule (I) :

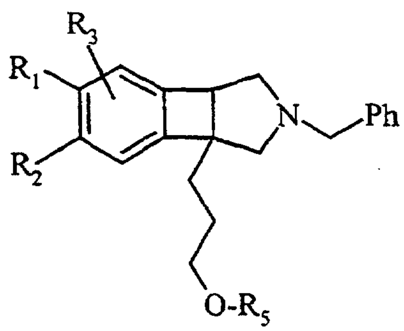

**(I/i)**

dans laquelle $R_1$, $R_2$, $R_3$, et $R_5$ sont tels que définis précédemment,
composés de formule **(I/i)** qui peuvent être débenzylés par une des méthodes classiquement utilisées en synthèse organique pour conduire aux composés de formule **(I/j),** à jonction de cycle cis, cas particulier des composés de formule (I) :

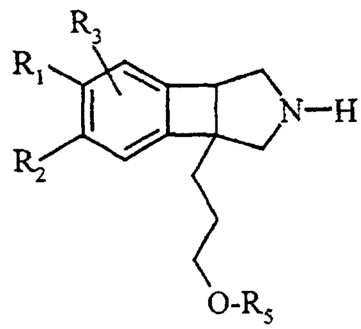

(I/j)

dans laquelle $R_1$, $R_2$, $R_3$, et $R_5$ sont tels que définis précédemment, composés de formule **(I/j)** qui peuvent être :

* soit transformés par traitement par le formaldéhyde en présence de cyanoborohydrure de sodium en composés de formule **(I/k),** à jonction de cycle *cis,* cas particulier des composés de formule (I) :

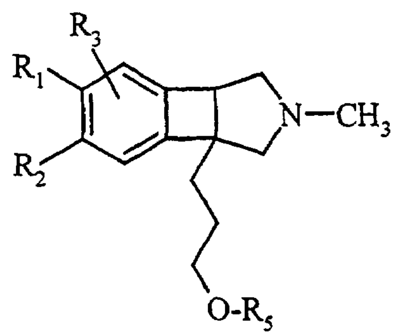

(I/k)

dans laquelle $R_1$, $R_2$, $R_3$ et $R_5$ sont tels que définis précédemment,

* soit mis en présence, selon des conditions classiques de synthèse organique, d'un composé de formule **(IX) :**

$$R'_4 - Z \qquad \textbf{(IX)}$$

dans lequel $R'_4$ a les mêmes définitions que $R_4$ à l'exception de hydrogène et benzyle, et Z représente un groupement partant usuellement utilisé en synthèse organique, pour conduire aux composés de formule (**I/l**), à jonction de cycle *cis,* cas particulier des composés de formule (I) :

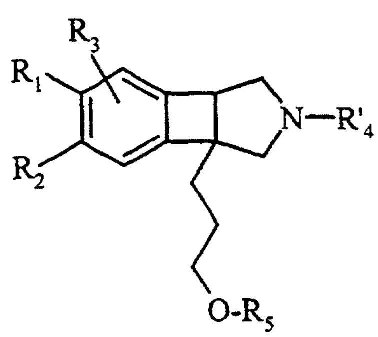

(I/l)

dans laquelle $R_1$, $R_2$, $R_3$, $R'_4$ et $R_5$ sont tels que définis précédemment,

les composés **(I/a)** à **(I/l)** formant l'ensemble des composés de l'invention, que l'on purifie, le cas échéant, selon une technique classique de purification, qui peuvent si on le désire, être séparés en leurs différents isomères selon une technique classique de séparation et que l'on transforme, le cas échéant, en leurs sels

d'addition à un acide ou à une base pharmaceutiquement acceptable.

Les composés de formule **(II)** sont des produits obtenus à partir de substances connues, selon des procédés connus, comme décrit ci-après dans les préparations 1 à 9. Ces préparations sont présentées ici à titre indicatif et non limitatif.

Les composés de formule **(V), (VIII)** et **(IX)** sont soit des composés commerciaux, soit obtenus selon les méthodes classiques de synthèse organique.

Les composés de la présente invention constituent des inhibiteurs de la recapture de la sérotonine et de par cette propriété caractéristique sont utiles pour le traitement de la dépression, des attaques de panique, des troubles obsessionnels compulsifs, des phobies, des troubles impulsifs, de l'abus de drogue et de l'anxiété.

A ce titre, la présente invention a également pour objet les compositions pharmaceutiques renfermant comme principe actif au moins un composé de formule (I), ses isomères optiques ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les sachets, les gélules, les tablettes, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature et la sévérité de l'affection et la prise de traitements éventuels associés et s'échelonne de 0,5 mg à 50 mg en une ou plusieurs prises par jour.

Les exemples suivant illustrent l'invention mais ne la limitent en aucune façon.

Les produits de départ utilisés sont des produits connus ou préparés selon des modes opératoires connus ou des préparations décrites (préparations 1 à 9).

Les différents stades de synthèse conduisent à des intermédiaires de synthèse, utiles pour la préparation des composés de l'invention.

Les structures des composés décrits dans les préparations, dans les stades de synthèses et dans les exemples ont été déterminées selon les techniques spectrophotométriques usuelles (infrarouge, RMN, spectromètre de masse, ...).

Les points de fusion ont été déterminés soit à la platine chauffante de Kôfler (K) soit à la platine chauffante sous microscope (MK).

**PREPARATION 1:**

**4-(2-Bromophényl)3-cyano-N-benzylpyrrolidine**

***Stade 1 : 3-{Benzyl[2-(2-bromophényl)-2-oxoéthyl]amino}propanenitrile***

Sur une solution de 61,7 g de N-benzyl 2-cyano éthylamine et de 67 ml de diisopropyléthylamine dans 200 ml d'acétone, on coule goutte à goutte à température ambiante une solution de 97,5 g de 2-bromo $\alpha$-bromoacétophénone dans 200 ml d'acétone. Après 12 heures d'agitation, le milieu réactionnel est évaporé, repris par de l'éther, lavé deux fois par 500 ml d'eau et séché. Après évaporation sous pression réduite, on obtient 131,9 g de produit attendu.

***Stade 2 : 3-{Benzyl[2-(2-bromophényl)-2-hydroxyéthyl]amino}propanenitrile***

Les 131,9 g de produit obtenus dans le stade 1 sont dissous dans 300 ml de méthanol puis additionnés goutte à goutte sur 39,9 g de borohydrure de sodium dans 300 ml de méthanol. La température est maintenue à 0°C pendant toute la durée de l'addition, puis le milieu réactionnel est abandonné à température ambiante pendant une nuit, puis évaporé à sec, repris avec du dichlorométhane et lavé à l'eau. Après concentration sous pression réduite, une chromatographie sur gel de silice (dichlorométhane) permet d'isoler 94 g de produit attendu.

***Stade 3 : 3-{Benzyl[2-(2-bromophényl)-2-chloroéthyl]amino}propanenitrile***

Les 94 g de produit obtenus au stade 2 sont mis en solution dans 400 ml de chlorure de méthylène. A température ambiante, en 1 heure, on coule goutte à goutte 90,5 ml de chlorure de thionyle. Après 12 heures sous agitation à température ambiante, le milieu réactionnel est évaporé à sec puis repris par de l'éther et filtré. Le solide est repris par 500 ml d'une solution aqueuse de carbonate de sodium à 10 % et par 1 1 de chlorure de méthylène. Après décantation, séchage et évaporation sous pression réduite, on obtient 90,2 g de produit attendu.

***Stade 4* : 4-(2-Bromophényl)-3-cyano-N-benzylpyrrolidine**

Les 90,2 g de produit obtenus au stade 3 sont mis en solution dans le tétrahydrofurane. On refroidit à -70°C et coule goutte à goutte 143 ml d'une solution 2M dans le tétrahydrofurane de sodium hexaméthyldisilazide. Après 1 heure à -70°C puis 12 heures à température ambiante, le milieu réactionnel est versé sur 300 ml d'une solution aqueuse saturée de chlorure d'ammonium, puis extrait à l'éther. Les phases organiques jointes sont lavées et séchées. Après évaporation on obtient 78,5 g de produit attendu sous forme d'un mélange de diastéréoisomères.

**PREPARATION 2 :**

**4-(2-Bromo-4-méthoxyphényl)-3-cyano-N-benzylpyrrolidine**

Sur 24 g d'un mélange E/Z (70 % / 30 %) de 2-bromo-4-méthoxy-cinnamonitrile, dissous dans 200 ml de dichlorométhane, en présence de quelques gouttes d'acide trifluoroacétique, est introduite goutte à goutte, une solution de N-butoxyméthyl-N-triméthysilylméthyl benzylamine dans 50 ml de dichlorométhane. La température augmente jusqu'à 40°C. On maintient à cette température pendant 1 heure 30. On ajoute après retour à température ambiante, 10 g de $K_2CO_3$ et laisse encore agiter 30 minutes. Après filtration et concentration sous pression réduite, une chromatographie sur gel de silice (dichlorométhane), permet d'isoler 26 g du produit attendu sous forme d'un mélange de diastéréoisomères dans une proportion 70 % / 30 %.

**PREPARATION 3 :**

**4-(2-Chloro-5-trifluorométhylphényl)-3-cyano-N-benzylpyrrolidine**

Le produit est obtenu suivant le procédé décrit dans la préparation 2 en utilisant comme substrat le 2-chloro-5-trifluorométhyl-cinnamonitrile.

**PREPARATION 4 :**

**4-(2-Bromo-5-fluorophényl)-3-cyano-N-benzylpyrrolidine**

Le produit est obtenu suivant le procédé décrit dans la préparation 2 en utilisant comme substrat le 2-bromo-5-fluoro-cinnamonitrile.

**PREPARATION 5 :**

**4-(2-Chloro-6-fluorophényl)-3-cyano-N-benzylpyrrolidine**

Le produit est obtenu selon le procédé décrit dans la préparation 2, en utilisant comme substrat le 2-chloro-6-fluoro-cinnamonitrile.

**PREPARATION 6 :**

**4-(2-Chloro-4-fluorophényl)-3-cyano-N-benzylpyrrolidine**

Le produit est obtenu selon le procédé décrit dans la préparation 2, en utilisant comme substrat le 2-chloro-4-fluoro-cinnamonitrile.

**PREPARATION 7 :**

**4-(2-Bromo-4,5-diméthoxyphényl)-3-cyano-N-benzylpyrrolidine**

Le produit est obtenu selon le procédé décrit dans la préparation 2, en utilisant comme substrat le 2-bromo-4,5-diméthoxy-cinnamonitrile.

**PREPARATION 8 :**

**4-(2-Chloro-4,5-méthylènedioxyphényl)-3-cyano-N-benzylpyrrolidine**

Le produit est obtenu selon le procédé décrit dans la préparation 2, en utilisant comme substrat le 2-chloro-4,5-méthylènedioxy-cinnamonitrile.

**PREPARATION 9 :**

**4-(2-Chloro-5-méthoxyphényl)-3-cyano-N-benzylpyrrolidine**

Le produit est obtenu selon le procédé décrit dans la préparation 2, en utilisant comme substrat le 2-chloro-5-méthoxy-cinnamonitrile.

**Exemple 1 :**

***cis*-3a-[(3,4-Méthylènedioxyphényloxy)méthyl]-2,3,3a,7b-tétrahydro-1*H*-benzo[3,4] cyclobuta[1,2-c]pyrrole et son chlorhydrate**

**Stade A : *cis*-2-Benzyl-2,3,3a,7b-tétrahydro-1*H*-benzo[3,4]cyclobuta[1,2-c]pyrrole-3a-carboximidamide et son nitrate**

78,4 g du produit de la préparation 1 dissous dans 50 ml d'éther sont introduits goutte à goutte dans de l'amidure de sodium dans 1,151 d'ammoniac liquide. Après 3 heures de contact on ajoute au milieu réactionnel 75 g de nitrate d'ammonium, puis on laisse s'évaporer l'ammoniac. On reprend par du chlorure de méthylène et de l'eau, sépare les deux phases, sèche la phase organique sur sulfate de magnésium et évapore le solvant. Après trituration à l'éther et concrétisation à l'acétate d'éthyle on obtient 42,2 g de produit attendu sous forme de son nitrate.
*Point de fusion : 183-187°C (M.K.).*

**Stade B : *cis*-2-Benzyl-2,3,3a,7b-tétrahydro-1*H*-benzo[3,4]cyclobuta[1,2-c]pyrrole-3a-carboxamide**

10,2 g du produit obtenu au stade A sont mis en suspension à température ambiante dans 90 ml d'éthanol. On additionne successivement 30 ml de soude 1N puis 30 ml d'eau. On porte 2 heures à reflux, puis évapore à sec. On reprend au chlorure de méthylène, lave par 200 ml d'une solution aqueuse saturée de bicarbonate de sodium et sèche sur sulfate de magnésium. Après évaporation on obtient 8,15 g de produit attendu.

**Stade C : cis-2-Benzyl-3a-méthyloxycarbonyle-2,3,3a,7b-tétrahydro-1*H*-benzo[3,4] cyclobuta[1,2-c]pyrrole**

8,10 g du produit obtenu au stade B sont mis en solution dans 90 ml de méthanol. On ajoute 11,6 ml de N, N-diméthylformamide diméthyl acétal puis abandonne la nuit sous agitation. On ajoute alors une solution d'éthanol chlorhydrique et on évapore à sec. On reprend à l'eau et lave à l'acétate d'éthyle. On basifie la phase aqueuse par du carbonate de sodium puis extrait au chlorure de méthylène. Après séchage et évaporation on obtient 8,1 g de produit attendu.

**Stade D : *cis*-2-Benzyl-3a-hydroxyméthyl-2,3,3a,7b-tétrahydro-1*H*-benzo[3,4] cyclobuta[1,2-c]pyrrole**

On ajoute goutte à goutte une solution de 4 g de composé obtenu au stade C dans 50 ml de tétrahydrofurane sur une suspension de 1,03 g de $LiAlH_4$ dans 15 ml de tétrahydrofurane. On laisse la nuit à température ambiante. Après hydrolyse et filtration des sels, on évapore à sec pour obtenir 3,5 g de produit attendu.

**Stade E : *cis*-2-Benzyl-3a-[(3,4-méthylènedioxyphényloxy)méthyl]-2,3,3a,7b-tétrahydro-1*H*-benzo[3,4]cyclobuta[1,2-c]pyrrole**

Dans un tricol on mélange 3,5 g du composé obtenu au stade D, 2g de 3,4-méthylènedioxyphénol, 3,8 g de triphénylphosphine dans 40 ml de tétrahydrofurane. On refroidit à 0°C et ajoute goutte à goutte, en 20 minutes, 2,4 ml de diéthylazodicarboxylate. Après 4 jours de réaction à température ambiante, le milieu réactionnel est concentré sous pression réduite. Une chromatographie sur gel de silice ($CH_2Cl_2$/AcOEt : 95/5) permet d'isoler 4,4 g de produit attendu.

**<u>Stade F</u> : *cis*-3a-[(3,4-Méthylènedioxyphényloxy)méthyl]-2,3,3a,7b-tétrahydro-1H-benzo[3,4]cyclobuta[1,2-c] pyrrole et son chlorhydrate**

Dans 4,4 g du produit obtenu au stade E, dissous dans 30 ml de dichloroéthane, on ajoute 2,5 ml de 1-chloroéthylchloroformiate et porte 3 heures à reflux. On évapore à sec, reprend avec 30 ml de méthanol et porte à reflux une nuit. Après évaporation, une chromatographie sur gel de silice permet d'isoler 1,45 g de produit attendu dont on prépare le chlorhydrate.
*<u>Point de fusion</u> (chlorhydrate) : 208-213°C (M.K.)*

**<u>Exemple 2</u> :**

***cis*-2-Benzyl-5-méthoxy-3a-[(3,4-méthylènedioxyphényloxy)-méthyl]-2,3,3a,7b-tétrahydro-1*H*-benzo[3,4]cyclobuta[1,2-c]pyrrole**

On procède comme dans l'exemple 1, des stades A à E, en utilisant comme substrat au stade A, le produit de la préparation 2.
*<u>Point de fusion</u> : 107-109°C (M.K).*

**<u>Exemple 3</u>:**

***cis*-5-Méthoxy-3a-[(3,4-méthylènedioxyphényloxy)-méthyl]-2,3,3a,7b-tétrahydro-1*H*-benzo[3,4]cyclobuta [1,2-c]pyrrole et son chlorhydrate**

On procède comme dans le stade F de l'exemple 1 à partir du produit obtenu dans l'exemple 2.
*<u>Point de fusion</u> (chlorhydrate) : 218-223°C (M.K.)*

**<u>Exemple 4</u> :**

***cis*-5-Méthoxy-2-méthyl-3a-[(3,4-méthylènedioxyphényloxy)-méthyl]-2,3,3a,7b-tétrahydro-1*H*-benzo[3,4]cyclobuta[1,2-c]pyrrole**

0,75 g du produit obtenu dans l'exemple 3 sous forme de base libre est mis en solution dans 25 ml de méthanol et 1,35 g d'acide acétique. On ajoute 0,6 ml de formaldéhyde à 37 % dans l'eau, puis 0,25 g de cyanoborohydrure de sodium. Après 2 jours de réaction à température ambiante, le milieu réactionnel est concentré, repris à l'eau, basifié à la soude, extrait à l'acétate d'éthyle, lavé à l'eau et à la saumure, séché et concentré. Une recristallisation de l'éthanol permet d'isoler 0,42 g de produit attendu. *<u>Point de fusion</u> : 94-96°C (M.K.)*

**<u>Exemple 5</u> :**

***cis*-6-Trifluorométhyl-3a-[(3,4-méthylènedioxyphényloxy)-méthyl]-2,3,3a,7b-tétrahydro-1*H*-benzo[3,4]cyclobuta[1,2-c]pyrrole et son chlorhydrate**

On procède comme dans l'exemple 1, des stades A à F, en utilisant comme substrat au stade A le produit de la préparation 3.
*<u>Point de fusion</u> (chlorhydrate) : 186-190°C (M.K.)*

**<u>Exemple 6</u> :**

***cis*-6-Fluoro-3a-[(3,4-méthylènedioxyphényloxy)-méthyl]-2,3,3a,7b-tétrahydro-1*H*-benzo[3,4]cyclobuta[1,2-c] pyrrole et son chlorhydrate**

On procède comme dans l'exemple 1, des stades A à F, en utilisant comme substrat au stade A le produit de la préparation 4.
*<u>Point de fusion</u> (chlorhydrate) : 248-252°C (M.K.)*

**Exemple 7 :**

***cis*-6-Fluoro-2-méthyl-3a-[(3,4-méthylènedioxyphényloxy)-méthyl]-2,3,3a,7b-tétrahydro-1*H*-benzo[3,4]cyclobuta[1,2-c]pyrrole et son chlorhydrate**

On procède comme dans l'exemple 4 en utilisant comme substrat le produit de l'exemple 6.
*Point de fusion : 212-216°C*

| *Microanalyse élémentaire* | | | | |
|---|---|---|---|---|
| | *C* | *H* | *N* | *Cl* |
| *% trouvé* | *62,66* | *5,40* | *3,90* | *10,16* |
| *% calculé* | *62,72* | *5,26* | *3,85* | *9,74* |

**Exemple 8 :**

***cis*-7-Fluoro-3a-[(3,4-méthylènedioxyphénoxy)-méthyl]-2,3,3a,7b-tétrahydro-1*H*-benzo[3,4]cyclobuta[1,2-c] pyrrole et son chlorhydrate**

On procède comme dans l'exemple 1 des stades A à F, en utilisant comme substrat au stade A le produit de la préparation 5.
*Point de fusion (M.K.) : 215-219°C*

| *Microanalyse élémentaire :* | | | | |
|---|---|---|---|---|
| | *C* | *H* | *N* | *Cl* |
| *% trouvé* | *61,94* | *5,16* | *3,94* | *10,29* |
| *% calculé* | *61,81* | *4,90* | *4,00* | *10,14* |

**Exemple 9 :**

***cis*-5-Fluoro-3a-[(3,4-méthylénedioxyphénoxy)-méthyl]-2,3,3a,7b-tétrahydro-1*H*-benzo[3,4]cyclobuta[1,2-c] pyrrole et son chlorhydrate**

On procède comme dans l'exemple 1 des stades A à F, en utilisant comme substrat au stade A le produit de la préparation 6.
*Point de fusion (M.K.) : 196-198°C*

| *Microananalyse élémentaire :* | | | | |
|---|---|---|---|---|
| | *C* | *H* | *N* | *Cl* |
| *% trouvé* | *61,60* | *5,12* | *4,01* | *10,61* |
| *% calculé* | *61,81* | *4,90* | *4,00* | *10,14* |

**Exemple 10 :**

***cis*-5,6-Diméthoxy-3a-[(3,4-méthylènedioxyphénoxy)-méthyl]2,3,3a,7b-tétrahydro-1*H*-benzo[3,4]cyclobuta [1,2-c]pyrrole et son chlorhydrate**

On procède comme dans l'exemple 1 des stades A à F, en utilisant comme substrat au stade A le produit de la préparation 7.
*Point de fusion (M.K.) : 202-206°C*

| *Microanalyse élémentaire :* | | | | |
|---|---|---|---|---|
| | *C* | *H* | *N* | *Cl* |
| *% trouvé* | *61,17* | *5,69* | *4,01* | *8,97* |
| *% calculé* | *61,30* | *5,66* | *3,57* | *9,05* |

**Exemple 11 :**

***cis*-5,6-Méthylènedioxy-3a-[(3,4-méthylènedioxyphénoxy)-méthyl]-2,3,3a,7b-tétrahydro-1*H*-benzo[3,4]cyclobuta[1,2-c]pyrrole et son chlorhydrate**

On procède comme dans l'exemple 1 des stades A à F, en utilisant comme substrat au stade A le produit de la préparation 8.
*Point de fusion (M.K.) : 212-214°C*

| *Microanalyse élémentaire :* | | | | |
|---|---|---|---|---|
| | *C* | *H* | *N* | *Cl* |
| *% trouvé* | *60,53* | *4,98* | *4,10* | *9,04* |
| *% calculé* | *60,73* | *4,83* | *3,73* | *9,43* |

**Exemple 12 :**

**6-Méthoxy-3a-[(3,4-méthylènedioxyphénoxy)-méthyl]-2,3,3a,7b-tétrahydro-1*H*-benzo[3,4]cyclobuta[1,2-c]pyrrole et son chlorhydrate**

On procède comme dans l'exemple I des stades A à F, en utilisant comme substrat au stade A le produit de la préparation 9.
*Point de fusion (M.K.) : 162-164°C*

| *Microanalyse élémentaire :* | | | | |
|---|---|---|---|---|
| | *C* | *H* | *N* | *Cl* |
| *% trouvé* | *63,14* | *5,51* | *3,97* | *10,07* |
| *% calculé* | *63,07* | *5,57* | *3,87* | *9,80* |

**Exemple 13 :**

***cis*-3a-[(2,1,3-Benzoxadiazol-5-yloxy)méthyl]-2,3,3a,7b-tétrahydro-1*H*-benzo[3,4]cyclobuta[1,2-c]pyrrole et son chlorhydrate**

On procède comme dans l'exemple 1 des stades A à F, en utilisant au stade E de cet exemple le 2,1,3-benzoxadiazol-5-ol à la place du 3,4-méthylènedioxyphénol.
*Point de fusion : 235-237°C*

| *Microanalyse élémentaire :* | | | | |
|---|---|---|---|---|
| | *C* | *H* | *N* | *Cl* |
| *% trouvé* | *61,55* | *5,00* | *12,46* | *10,82* |
| *% calculé* | *61,92* | *4,89* | *12,74* | *10,75* |

**Exemple 14 :**

***cis*-3a-[(4-Cyclopentylphénoxy)méthyl]-2,3,3a,7b-tétrahydro-1*H*-benzo[3,4]cyclobuta [1,2-c]pyrrole**

On procède comme dans l'exemple 1 des stades A à F, en utilisant au stade E de cet exemple le 4-cyclopentylphénol à la place du 3,4-méthylènedioxyphénol.
*Point de fusion : 80-82°C*

| *Microanalyse élémentaire :* | | | |
|---|---|---|---|
| | *C* | *H* | *N* |
| *% trouvé* | *82,05* | *7,74* | *4,36* |
| *% calculé* | *82,72* | *7,89* | *4,38* |

**Exemple 15 :**

***cis*-3a-{[(Thiochroman-4-one-6-yl)oxy]méthyl}-2,3,3a,7b-tétrahydro-1*H*-benzo[3,4] cyclobuta[1,2-c]pyrrole et son chlorhydrate**

On procède comme dans l'exemple 1 des stades A à F, en utilisant au stade E de cet exemple le 6-hydroxythiochromane-4-one à la place du 3,4-méthylènedioxyphénol.
*Point de fusion : 211-213°C*

| *Microanalyse élémentaire :* | | | | | |
|---|---|---|---|---|---|
| | *C* | *H* | *N* | *Cl* | *S* |
| *% trouvé* | *64,44* | *5,50* | *3,96* | *9,24* | *8,32* |
| *% calculé* | *64,25* | *5,39* | *3,75* | *9,48* | *8,58* |

**Exemple 16 :**

***cis*-3a-{[4-(1-Adamantyl)phénoxy]méthyl}-2,3,3a,7b-tétrahydro-1*H*-benzo[3,4]cyclobuta[1,2-c]pyrrole et son chlorhydrate**

On procède comme dans l'exemple 1 des stades A à F, en utilisant au stade E de cet exemple le 4-(1-adamantyl)phénol à la place du 3,4-méthylènedioxyphénol.
*Point de fusion (M.K.) : 244-248°C*

| *Microanalyse élémentaire :* | | | | |
|---|---|---|---|---|
| | *C* | *H* | *N* | *Cl* |
| *% trouvé* | *77,37* | *7,73* | *3,52* | *8,77* |
| *% calculé* | *76,85* | *7,64* | *3,32* | *8,40* |

**Exemple 17 :**

***cis*-3a-[(5-Indanyloxy)méthyl]-2,3,3a,7b-tétrahydro-1*H*-benzo[3,4]cyclobuta[1,2-c] pyrrole et son chlorhydrate**

On procède comme dans l'exemple 1 des stades A à F, en utilisant au stade E de cet exemple le 5-hydroxyindane à la place du 3,4-méthylènedioxyphénol.
*Point de fusion (M.K.) : 224-227°C*

| *Microanalyse élémentaire:* | | | | |
|---|---|---|---|---|
| | *C* | *H* | *N* | *Cl* |
| *% trouvé* | *73,17* | *6,92* | *4,43* | *11,19* |
| *% calculé* | *73,27* | *6,76* | *4,27* | *10,81* |

**Exemple 18 :**

***cis*-3a-{[(4-Méthyl-2*H*-chromène-2-one-7-yloxy]méthyl}-2,3,3a,7b-tétrahydro-1*H*-benzo[3,4]cyclobuta[1,2-c] pyrrole et son chlorhydrate**

On procède comme dans l'exemple 1 des stades A à F, en utilisant au stade E de cet exemple le 7-hydroxy-4-méthyl-chromèn-2-one à la place du 3,4-méthylènedioxyphénol.
*Point de fusion (M.K.) : 217-222°C*

| *Microanalyse élémentaire :* | | | | |
|---|---|---|---|---|
| | *C* | *H* | *N* | *Cl* |
| *% trouvé* | *68,38* | *5,85* | *3,84* | *9,77* |
| *% calculé* | *68,20* | *5,45* | *3,79* | *9,59* |

**Exemple 19 :**

***cis*-3a-[(Chromèn-2-one-7-yloxy)méthyl]-2,3,3a,7b-tétrahydro-1*H*-benzo[3,4] cyclobuta [1,2-c]pyrrole et son chlorhydrate**

On procède comme dans l'exemple 1 des stades A à F, en utilisant au stade E de cet exemple le 7-hydroxy-chromèn-2-one à la place du 3,4-méthylènedioxyphénol.
*Point de fusion (M.K.) : 231-235°C*

| *Microanalyse élémentaire :* | | | | |
|---|---|---|---|---|
| | *C* | *H* | *N* | *Cl* |
| *% trouvé* | *67,38* | *5,61* | *3,98* | *10,49* |
| *% calculé* | *67,51* | *5,10* | *3,94* | *9,96* |

**Exemple 20 :**

***cis*-3a-[(2-Ethoxyphényloxy)méthyl]-2,3,3a,7b-tétrahydro-1*H*-benzo[3,4]cyclobuta [1,2-c]pyrrole et son chlorhydrate**

On procède comme dans l'exemple 1 des stades A à F, en utilisant au stade E de cet exemple le 2-éthoxyphénol à la place du 3,4-méthylènedioxyphénol.
*Point de fusion (M.K.) : 200-204°C*

| *Microanalyse élémentaire :* | | | | |
|---|---|---|---|---|
| | *C* | *H* | *N* | *Cl* |
| *% trouvé* | *68,74* | *6,91* | *4,32* | *10,89* |
| *% calculé* | *68,77* | *6,68* | *4,22* | *10,68* |

**Exemple 21 :**

***cis*-3a-[2-(3,4-Méthylènedioxyphényloxy)éthyl]-2,3,3a,7b-tétrahydro-1*H*-benzo[3,4]cyclobuta[1,2-c]pyrrole et son chlorhydrate**

**Stade A : *cis*-2-Benzyl-3a-bromométhyl-2,3,3a,7b-tétrahydro-1*H*-benzo[3,4] cyclobuta[1,2-c]pyrrole**

Un mélange composé de 5,3 g du produit obtenu au stade D de l'exemple 1, 9 g de tétrabromure de carbone et 7,2 g de triphénylphosphine dans 115 ml d'éther est porté à reflux pendant 24 heures. Après refroidissement, filtration du précipité, et concentration, le résidu obtenu est purifié par chromatographie sur gel de silice (dichlorométhane/ éthanol : 95/5) permettant d'isoler le produit attendu.

**Stade B : *cis*-2-Benzyl-3a-cyanométhyl-2,3,3a,7b-tétrahydro-1*H*-benzo[3,4] cyclobuta[1,2-c]pyrrole**

4,76 g du produit obtenu au stade A précédent est dissout dans 75 ml de tétrahydrofurane, puis 6,26 g de cyanure de tétrabutylammonium sont additionnés par portions. Après 12 heures à température ambiante, le milieu est dilué avec 75 ml d'éther puis est versé sur 25 ml d'une solution saturée de bicarbonate de sodium. Après décantation, lavage avec de l'acide chlorhydrique 1N, puis alcalinisation, les phases organiques extraites au dichlorométhane sont lavées, séchées, filtrées puis concentrées sous pression réduite permettant d'isoler le produit attendu.

**Stade C : Acide *cis*(2-benzyl-2,3,3a,7b-tétrahydro-1*H*-benzo[3,4]cyclobuta[1,2-c] pyrrol-3a-yl)acétique**

Un mélange composé de 2,64 g du composé obtenu au stade B et de 31 ml d'acide chlorhydrique à 20 % est chauffé à reflux pendant 4 heures, puis 31 ml d'acide chlorhydrique à 20 % sont ajoutés et le reflux est maintenu pendant 6 heures 30. Le milieu est évaporé permettant d'isoler le produit attendu qui est utilisé tel quel dans le stade suivant.

**Stade D : *cis*-2-(2-Benzyl-2,3,3a,7b-tétrahydro-1*H*-benzo[3,4]cyclobuta[1,2-c] pyrrol-3a-yl)éthanol**

3,32 g du produit obtenu au stade C sont introduits par portions sur une suspension composée de 0,73 g d'hydrure de lithium et d'aluminium et de 53 ml de tétrahydrofurane. Après 2 heures à température ambiante, le milieu est hydrolysé à froid et le précipité formé est filtré. Après évaporation du solvant, on isole le produit attendu.

**Stade E : *cis*-2-Benzyl-3a-[2-(3,4-méthylènedioxyphényloxy)-éthyl]-2,3,3a,7b-tétrahydro-1*H*-benzo[3,4]cyclobuta[1,2-c]pyrrole**

On procède comme au stade E de l'exemple 1, en utilisant comme substrat le produit du stade D obtenu ci-dessus.

**Stade F : *cis*-3a-[2-(3,4-Méthylènedioxyphényloxy)éthyl]-2,3,3a,7b-tétrahydro-1*H*-benzo[3,4]cyclobuta[1,2-c] pyrrole et son chlorhydrate**

On procède comme au stade F de l'exemple 1, en utilisant comme substrat le produit obtenu au stade E précédent.
*Point de fusion : 204-207°C*

| *Microanaalyse élémentaire :* | | | | |
|---|---|---|---|---|
| | *C* | *H* | *N* | *Cl* |
| *% trouvé* | *65,94* | *5,81* | *4,04* | *10,73* |
| *% calculé* | *65,99* | *5,83* | *4,05* | *10,23* |

**Exemple 22 :**

***cis*-3a-[(1-cyanobenzocyclobutan-5-yloxy)méthyl]-2,3,3a,7b-tétrahydro-1*H*-benzo[3,4] cyclobuta [1,2-c]pyrrole et son chlorhydrate**

On procède comme dans l'exemple 1 des stades A à F, en utilisant au stade E de cet exemple le 1-cyano-benzocyclobutan-5-ol à la place du 3,4-méthylènedioxyphénol.
*Point de fusion (M.K.) : 136-138°C*

**Exemple 23 :**

***cis*-3a-[(3,5-bis-trifluorométhylphenyloxy)méthyl]-2,3,3a,7b-tétrahydro-1*H*-benzo[3,4]cyclobuta [1,2-c]pyrrole et son chlorhydrate**

On procède comme dans l'exemple 1 des stades A à F, en utilisant au stade E de cet exemple le 3,5-bis-trifluorométhylphénol à la place du 3,4-méthylènedioxyphénol.
*Point de fusion (M.K.) : 242-244°C*

**Exemple 24 :**

***cis*-3a-[(2,3-dihydro-benzofuran-5-yloxy)méthyl]-2,3,3a,7b-tétrahydro-1*H*-benzo[3,4] cyclobuta [1,2-c]pyrrole et son chlorhydrate**

On procède comme dans l'exemple 1 des stades A à F, en utilisant au stade E de cet exemple le 2,3-dihydro-benzofuran-5-ol à la place du 3,4-méthylènedioxyphénol.
*Point de fusion (M.K.) : 208° C (décomposition)*

**Exemple 25 :**

***cis*-3a-[(2,3-dihydro-benzo[1,4]dioxan-6-yloxy)méthyl]-2,3,3a,7b-tétrahydro-1*H*-benzo[3,4]cyclobuta [1,2-c] pyrrole et son chlorhydrate**

On procède comme dans l'exemple 1 des stades A à F, en utilisant au stade E de cet exemple le 2,3-dihydro-benzo[1,4]dioxan-6-ol à la place du 3,4-méthylènedioxyphénol.
*Point de fusion (M.K.) : 219-222° C*

**Exemple 26 :**

***cis*-3a-[(3,4-diméthylphényloxy)méthyl]-2,3,3a,7b-tétrahydro-1*H*-benzo[3,4] cyclobuta [1,2-c]pyrrole et son chlorhydrate**

On procède comme dans l'exemple 1 des stades A à F, en utilisant au stade E de cet exemple le 3,4-diméthylphénol à la place du 3,4-méthylènedioxyphénol.
*Point de fusion (M.K.) : 230-232° C*

**Exemple 27 :**

***cis*-3a-[(3,4-dihydro-2*H*-thiochromèn-7-yloxy)méthyl]-2,3,3a,7b-tétrahydro-1*H*-benzo[3,4] cyclobuta [1,2-c]pyrrole et son chlorhydrate**

On procède comme dans l'exemple 1 des stades A à F, en utilisant au stade E de cet exemple le 7-thiochromanol à la place du 3,4-méthylènedioxyphénol.
*Point de fusion (M.K.) : 248-250° C*

## Etude pharmacologique des composés de l'invention

### *A. Etude in vitro*

**Exemple 28 :**

**Détermination de l'affinité pour les sites de recapture de la sérotonine**

L'affinité a été déterminée par des expériences de compétition avec la $[^3H]$-paroxetine (NEN, Les Ulis, France). Les membranes sont préparées à partir de cortex frontal de rat et incubées en triple avec 1,0 nM de $[^3H]$-paroxetine et le ligand froid dans un volume final de 0,4 ml, pendant 2 heures à 25° C. Le tampon d'incubation contient 50 nM de TRIS-HCl (pH 7,4), 120 mM de NaCl et 5 mM de KCl. La fixation non-spécifique est déterminée avec 10 μM de citalopram. A la fin de l'incubation, le milieu d'incubation est filtré et lavé trois fois avec 5 ml de tampon refroidi. La radioactivité retenue sur les filtres est déterminée par comptage du liquide de scintillation. Les isothermes de binding sont analysées par régression non-linéaire pour déterminer les valeurs d'$IC_{50}$. Elles sont converties en constante de dissociation ($K_i$) par l'intermédiaire de l'équation de Cheng-Prusoff :

$$K_1 = IC_{50} / (1 + L / K_d)$$

dans laquelle L est la concentration de $[^3H]$-paroxetine et $K_d$ est la constante de dissociation de $[^3H]$-paroxetine pour le site de recapture de la sérotonine (0,13 nM). Les résultats sont exprimés en $pK_i$ (- log $K_i$).

Les composés de la présente invention montrent une très bonne affinité pour les sites de recapture de la sérotonine. A titre d'exemple le $pK_i$ du composé de l'exemple 1 est de 7,9.

***B. Etude in vivo***

**Exemple 29 :**

**Expérience de la microdialyse chez le rat**

Les rats sont anesthésiés au pentobarbital (60 mg/kg i.p.). Ils sont placés dans un appareil stéréotaxique de Kopf et le guide de la canule est implanté dans le cortex frontal cingulé suivant les coordonnées décrites comme suit dans l'atlas de Paxinos et Watson (1982): AP = + 2,2 ; L = ± 0,6 ; DV = - 0,2. Les rats sont mis en cage séparément et ne sont utilisés en dialyse que 5 jours plus tard. Le jour de la dialyse la sonde est descendue lentement et maintenue dans sa position. La sonde est perfusée à un débit de 1 µl/mn avec une solution de 147,2 mM de NaCl, 4 mM de KCl et 2,3 mM de $CaCl_2$ amené à pH 7,3 avec un tampon phosphate (0,1 M). Deux heures après l'implantation, les échantillons sont collectés toutes les 20 minutes pendant 4 heures. Trois échantillons de base sont collectés avant l'administration des produits à tester. Les rats sont laissés dans leur cage individuelle pendant toute l'expérience. A la fin de l'expérience, les rats sont décapités et le cerveau prélevé est congelé dans l'isopentane. Des sections d'une épaisseur de 100 µm sont coupées et colorées avec du cresyl violet, ce qui permet la vérification de l'emplacement des sondes. La quantification simultanée de dopamine, norépinéphrine et sérotonine est effectuée de la façon suivante : 20 µl d'échantillon de dialyse sont dilués avec 20 µl de phase mobile ($NaH_2PO_4$ : 75 mM, EDTA : 20 µM, sodium 1-decanesulfonate : 1 mM, méthanol : 17,5 %, triethylamine : 0,01 %, pH : 5,70) et 33 µl sont analysés par HPLC avec une colonne en phase inverse thermostatée à 45° C et quantifiés par l'intermédiaire d'un détecteur coulométrique. Le potentiel de la première électrode du détecteur est fixée à- 90 mV (réduction) et la seconde à + 280 mV (oxydation). La phase mobile est injectée avec une pompe à un débit de 2 ml/mn. Les limites de sensibilité pour la dopamine, la norépinéphrine et la sérotonine sont de 0,55 fmole par échantillon. Tous les produits de l'invention sont injectés par voie sous-cutanée dans un volume de 1,0 ml/kg. Les produits sont dissous dans de l'eau distillée additionnée de quelques gouttes d'acide lactique si nécessaire.

***Résultats :***

A titre d'exemple et pour illustrer l'activité des produits de l'invention, le composé de l'exemple 1, administré à la dose de 10 mg/kg, par voie sous-cutanée, augmente respectivement les niveaux de sérotonine, de dopamine et de noradrénaline de :
+ 226,3 % ± 20,1 ; + 54,8 % ± 6,4 et + 96,4 % ± 7,8 (% maximal de l'effet par rapport au niveau basal défini comme le 0 %).

**Exemple 30 :**

**Test d'agressivité chez des souris isolées**

Ce test permet d'évaluer l'activité anti-agressive *intraspecies* d'un produit chez des souris qui ont été maintenues en isolement pendant plusieurs mois.

*Animaux :*

Le test utilise des souris mâles CD (Charles River) de poids 22 à 25 g à leur arrivée dans l'animalerie. Dès leur arrivée, les animaux sont isolés dans des cages individuelles en polycarbonate opaque noir (23 x14 x13 cm) avec un couvercle grillagé et stabulés de façon chronique (6 mois environ) dans la pièce d'expérimentation.

*Sélection des couples de souris :*

La sélection des couples de souris agressives qui seront utilisés de façon chronique dans l'étude, commence après un mois d'isolement des animaux. Une ou deux fois par semaine, on place dans la cage d'une souris (résidente), une souris d'une autre cage (intruse) et l'on observe si les deux animaux s'attaquent (reniflements, poursuites, mordillements, morsures) pendant cet essai. A la fin de l'essai (durée maximale de 10 minutes), chaque souris est isolée à nouveau dans sa cage respective. Si il y a eu des attaques, le même couple sera testé à nouveau lors du prochain essai ; si il n'y a pas eu d'attaques, chaque souris de ce couple sera mise en présence d'une autre souris, lors de

l'essai suivant. On sélectionne ainsi, au cours d'essais successifs, à raison de 1 ou 2 essais par semaines, les couples définitifs de souris qui seront utilisés pour les expériences. La sélection des couples est basée sur la stabilité de la combativité des animaux d'un essai à l'autre, la faible latence de la première attaque et la fréquence et durée des attaques. Chez les couples ainsi sélectionnés, ces paramètres sont vérifiés chaque semaine au cours d'un essai rapide, sans traitement, deux jours avant le jour Test.

*Test :*

Le test a lieu une fois par semaine. Trente minutes avant leur mise en présence, les deux souris du couple reçoivent chacune le même traitement (produit ou solvant) et restent isolées dans leur cage respective. A T0 minute, la souris intruse est introduite dans la cage de la souris résidente pour une durée de 3 minutes. On note la latence (en sec.) de la première attaque, le nombre et la durée totale (en sec.) des attaques. On note aussi une inversion éventuelle de la dominance d'une souris par rapport à l'autre (en général, la souris résidente est la souris dominante).
A la fin du test, la souris intruse retourne dans sa cage ; les animaux restent en isolement jusqu'au prochain essai rapide et test, la semaine suivante.
La Dose Inhibitrice 50 du nombre ou de la durée des attaques est la dose de produit qui réduit de moitié la moyenne de chacune de ces valeurs, par rapport à celle obtenue respectivement dans le groupe contrôle.

*Résultats :*

A titre d'exemple et pour illustrer l'activité des produits de l'invention, la dose inhibitrice 50, pour le composé de l'exemple 1 est inférieur à 5 mg/kg i.p.

## Revendications

**1.** Composés de formule (I), à jonction de cycle *cis* :

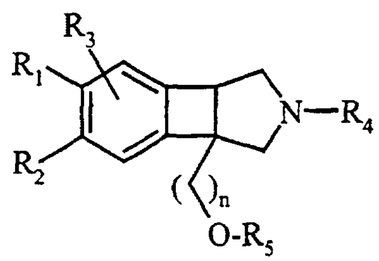

(I)

dans laquelle :

**$R_1$, $R_2$, $R_3$,** identiques ou différents, indépendamment l'un de l'autre, représentent un groupement choisi parmi atome d'hydrogène, d'halogène, groupement alkyle ($C_1$-$C_6$) linéaire ou ramifié, alkényle ($C_2$-$C_6$) linéaire ou ramifié, alkynyl ($C_2$-$C_6$) linéaire ou ramifié, hydroxy, alkoxy ($C_1$-$C_6$) linéaire ou ramifié, cycloalkyle, cycloalkylalkyle ($C_1$-$C_6$) linéaire ou ramifié, aryle, arylalkyle ($C_1$-$C_6$) linéaire ou ramifié, aryloxy, arylalkoxy ($C_1$-$C_6$) linéaire ou ramifié, trihalogénoalkyle ($C_1$-$C_6$) linéaire ou ramifié, trihalogénoalkoxy ($C_1$-$C_6$) linéaire ou ramifié, cyano, nitro, un groupement de formule $-OSO_2CH_3$, - $OSO_2CF_3$, $NR_6R_7$, $CO_2R_6$ dans lesquels $R_6$ et $R_7$, identiques ou différents, indépendamment l'un de l'autre, représentent un atome d'hydrogène, un groupement alkyle ($C_1$-$C_6$) linéaire ou ramifié, alkényle ($C_2$-$C_6$) linéaire ou ramifié, alkynyle ($C_2$-$C_6$) linéaire ou ramifié, cycloalkyle, cycloalkylalkyl ($C_1$-$C_6$) linéaire ou ramifié, aryle, arylalkyle ($C_1$-$C_6$) linéaire ou ramifié, ou $R_1$, $R_2$, $R_3$ pris par deux en positions adjacentes, représentent, avec les atomes communs du cycle benzénique auquel ils sont liés, un système cyclique ($C_4$-$C_8$), saturé ou insaturé, dont éventuellement un ou deux atomes de carbone est (sont) remplacé(s) par un ou deux hétéroatomes, identiques ou différents, choisis parmi oxygène, azote et soufre,
étant entendu que dans le cas où $R_1$, $R_2$, $R_3$ sont pris par deux en positions adjacentes et représentent la définition précédemment citée, alors le groupe restant $R_1$ ou $R_2$ ou $R_3$ prend l'une des définitions individuelles de ces groupements précédemment mentionnées,

**$R_4$** représente un groupement choisi parmi atome d'hydrogène, groupement alkyle ($C_1$-$C_6$) linéaire ou ramifié, alkényle ($C_2$-$C_6$) linéaire ou ramifié, alkynyle ($C_2$-$C_6$) linéaire ou ramifié, cycloalkyle, cycloalkylalkyle ($C_1$-$C_6$) linéaire ou ramifié, aryle, arylalkyle ($C_1$-$C_6$) linéaire ou ramifié, hétérocycloalkyle, hétérocycloalkylalkyle ($C_1$-$C_6$) linéaire ou ramifié, hétéroaryle et hétéroarylalkyle ($C_1$-$C_6$) linéaire ou ramifié,

**$R_5$** représente un groupement aryle, aryle éventuellement substitué, hétéroaryle ou hétéroaryle éventuellement substitué,

**n** représente un entier compris entre 1 et 3 inclus,

leurs isomères, ainsi que leurs sels d'addition à un acide ou à une base pharmaceutiquement acceptable,
étant entendu que par groupement cycloalkyle, on comprend un système cyclique ($C_3$-$C_8$), mono ou bicyclique, comportant éventuellement une ou plusieurs insaturation(s), ladite ou lesdites insaturations ne conférant pas un caractère aromatique au système cyclique, éventuellement substitué par un ou plusieurs groupements, identiques ou différents, choisis parmi atome d'halogène, et groupement alkyle ($C_1$-$C_6$) linéaire ou ramifié,
étant entendu que par groupement hétérocycloalkyle, on comprend un groupement cycloalkyle éventuellement substitué tel que défini précédemment et contenant au niveau du système cyclique un, deux ou trois hétéroatomes, identiques ou différents, choisis parmi oxygène, azote et soufre,
étant entendu aussi que par groupement aryle, on comprend un groupement phényle, naphtyle, dihydronaphtyle, tétrahydronaphtyle, indényle ou indanyle, et que par groupement aryle éventuellement substitué, on comprend un groupement aryle tel que défini éventuellement substitué par un ou plusieurs groupements, identiques ou différents, choisis parmi halogène, hydroxy, alkyle ($C_1$-$C_6$) linéaire ou ramifié, alkoxy ($C_1$-$C_6$) linéaire ou ramifié, alkényle ($C_1$-$C_6$) linéaire ou ramifié, cycloalkyle, adamantyle, cycloalkylalkyle ($C_1$-$C_6$) linéaire ou ramifié, aryle, arylalkyle ($C_1$-$C_6$) linéaire ou ramifié, aryloxy, arylalkoxy ($C_1$-$C_6$) linéaire ou ramifié, trihalogénoalkyle ($C_1$-$C_6$) linéaire ou ramifié, trihalogénoalkoxy ($C_1$-$C_6$) linéaire ou ramifié, cyano, nitro, oxo, un groupement de formule $-OSO_2CH_3$, $-OSO_2CF_3$, $NR_6R_7$, ou $CO_2R_6$ dans lesquels $R_6$ et $R_7$ sont tels que définis dans la formule (I),
étant entendu également que par groupement hétéroaryle, on comprend un groupement aryle tel que défini précédemment, contenant un, deux ou trois hétéroatomes, identiques ou différents, choisis parmi oxygène, azote et soufre, et que par groupement hétéroaryle éventuellement substitué, on comprend un groupement hétéroaryle tel que défini précédemment éventuellement substitué par un ou plusieurs groupements, identiques ou différents, tels que définis pour les substitutions des groupements aryles.

**2.** Composés de formule (I), selon la revendication 1 **caractérisés en ce que** $R_5$ représente un groupement hétéroaryle, leurs isomères ainsi que leurs sels d'addition à un acide ou à une base pharmaceutiquement acceptable.

**3.** Composés de formule (I) selon l'une quelconque des revendications 1 ou 2 **caractérisés en ce que** $R_5$ représente un groupement méthylènedioxyphényle ou éthylènedioxyphényle, leurs isomères ainsi que leurs sels d'addition à un acide ou à une base pharmaceutiquement acceptable.

**4.** Composés de formule (I) selon la revendication 1 **caractérisés en ce que** n est égal à 1, leurs isomères ainsi que leurs sels d'addition à un acide ou à une base pharmaceutiquement acceptable.

**5.** Composés de formule (I) selon la revendication 1 **caractérisés en ce que** n est égal à 2, leurs isomères ainsi que leurs sels d'addition à un acide ou à une base pharmaceutiquement acceptable.

**6.** Composés de formule (I) selon la revendication 1 **caractérisés en ce que** $R_4$ représente un atome d'hydrogène, un groupement alkyle ($C_1$-$C_6$) linéaire ou ramifié ou arylalkyle ($C_1$-$C_6$) linéaire ou ramifié, leurs isomères ainsi que leurs sels d'addition à un acide ou à une base pharmaceutiquement acceptable.

**7.** Composé de formule (I) selon la revendication 1 qui est le *cis*-3a-[(3,4-méthylènedioxyphényloxy)méthyl]-2,3,3a,7b-tétrahydro-1*H*-benzo[3,4]cyclobuta[1,2-c]pyrrole et son chlorhydrate.

**8.** Composé de formule (I) selon la revendication 1 qui est le *cis*-2-benzyl-5-méthoxy-3a-[(3,4-méthylènedioxyphényloxy)-méthyl]-2,3,3a,7b-tétrahydro-1*H*-benzo [3,4]cyclobuta[1,2-c]pyrrole.

**9.** Composé de formule (I) selon la revendication 1 qui est le *cis*-5-méthoxy-3a-[(3,4-méthylènedioxyphényloxy)-méthyl]-2,3,3a,7b-tétrahydro-1*H*-benzo [3,4]cyclobuta[1,2-c]pyrrole et son chlorhydrate.

**10.** Composé de formule (I) selon la revendication 1 qui est le *cis*-5-méthoxy-2-méthyl-3a-[(3,4-méthylènedioxyphényloxy)-méthyl]-2,3,3a,7b-tétrahydro-1*H*-benzo [3,4]cyclobuta[1,2-c]pyrrole.

**11.** Composé de formule (I) selon la revendication 1 qui est le *cis*-6-trifluorométhyl-3a-[(3,4-méthylènedioxyphényloxy)-méthyl]-2,3,3a,7b- tétrahydro-1*H*-benzo[3,4]cyclobuta[1,2-c]pyrrole et son chlorhydrate.

**12.** Composé de formule (I) selon la revendication 1 qui est le *cis*-6-fluoro-3a-[(3,4-méthylènedioxyphényloxy)-méthyl] 2,3,3a,7b-tétrahydro-1*H*-benzo [3,4]cyclobuta[1,2-c]pyrrole et son chlorhydrate.

**13.** Procédé de préparation des composés de formule (I) selon la revendication 1 **caractérisé en ce qu'**on utilise comme produit de départ un composé de formule (II) :

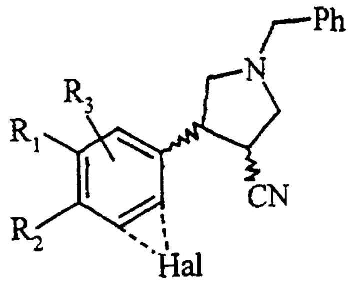

**(II)**

dans laquelle $R_1$, $R_2$, $R_3$ ont la même signification que dans la formule **(I)** et Hal représente un atome d'halogène, ledit atome d'halogène étant fixé sur l'un ou l'autre atome de carbone du cycle benzénique,
composés de formule **(II)** que l'on soumet à l'action de l'amidure de sodium dans l'ammoniaque liquide, pour conduire aux composés de formule **(III),** à jonction de cycle *cis*,

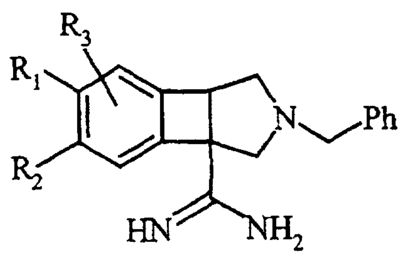

**(III)**

dans laquelle $R_1$, $R_2$, $R_3$ ont la même signification que dans la formule **(I),**
composés de formule **(III)** que l'on traite par une base minérale, en milieu alcoolique aqueux, pour conduire aux composés de formule **(IV),** à jonction de cycle *cis*,

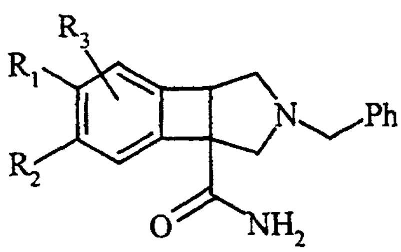

**(IV)**

dans laquelle $R_1$, $R_2$, $R_3$ ont la même signification que dans la formule **(I),**
composés de formule **(IV)** que l'on transforme par l'intermédiaire d'un acétal de N,N-diméthylformamide de formule **(V) :**

$$(CH_3)_2N\ CH(OG)_2 \qquad \textbf{(V)}$$

dans laquelle G représente un groupement alkyle ($C_1$-$C_6$) linéaire ou ramifié, benzyle ou cyclohexyle, en ester de formule **(VI),** à jonction de cycle *cis*,

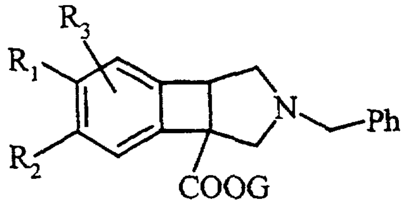

**(VI)**

dans laquelle $R_1$, $R_2$, $R_3$, et G sont tels que définis précédemment, composés de formule **(VI)** qui sont réduits, selon des conditions classiques de synthèse organique, en alcool de formule (**VII**), à jonction de cycle *cis*,

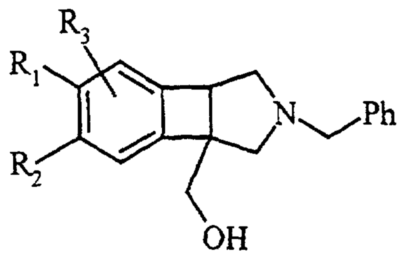

**(VII)**

dans laquelle $R_1$, $R_2$, $R_3$ sont tels que définis précédemment, composés de formule **(VII)** qui sont :

→ soit traités par un composé de formule **(VIII) :**

$R_5$ - OH **(VIII)**

dans laquelle $R_5$ a la même signification que dans la formule **(I),** pour conduire aux composés de formule **(I/a),** à jonction de cycle *cis*, cas particulier des composés de formule (I) :

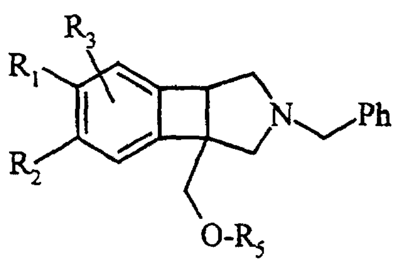

**(I/a)**

dans laquelle $R_1$, $R_2$, $R_3$ et $R_5$ sont tels que définis précédemment, composés de formule **(I/a)** qui peuvent être débenzylés par une des méthodes classiquement utilisées en synthèse organique, pour conduire aux composés de formule **(I/b),** à jonction de cycle *cis*, cas particulier des composés de formule (I) :

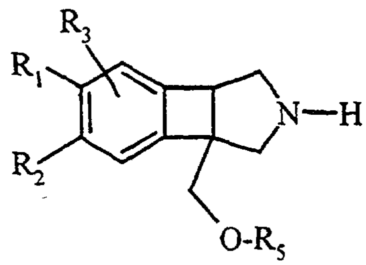

**(I/b)**

dans laquelle $R_1$, $R_2$, $R_3$ et $R_5$ sont tels que définis précédemment, composés de formule **(I/b)** qui peuvent être

* soit transformés par traitement par le formaldéhyde en présence de cyanoborohydrure de sodium en composés de formule **(I/c),** à jonction de cycle *cis*, cas particulier des composés de formule (I) :

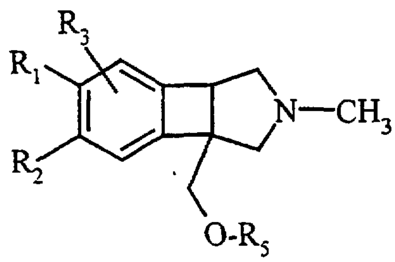

**(I/c)**

dans laquelle $R_1$, $R_2$, $R_3$ et $R_5$ sont tels que définis précédemment,

* soit mis en présence, selon des conditions classiques de synthèse organique, d'un composé de formule **(IX) :**

$$R'_4 - Z \qquad \textbf{(IX)}$$

dans lequel $R'_4$ a les mêmes définitions que $R_4$ à l'exception de hydrogène et benzyle, et Z représente un groupement partant usuellement utilisé en synthèse organique,
pour conduire aux composés de formule **(I/d),** à jonction de cycle *cis*, cas particulier des composés de formule (I) :

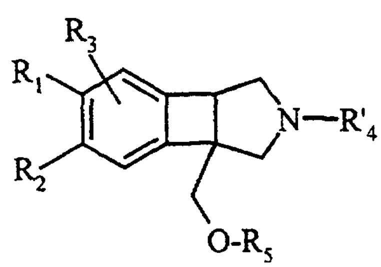

**(I/d)**

dans laquelle $R_1$, $R_2$, $R_3$, $R'_4$ et $R_5$ sont tels que définis précédemment,

→ soit transformés, par une suite de réactions classiques, en composés de formule **(X),** à jonction de cycle *cis*,

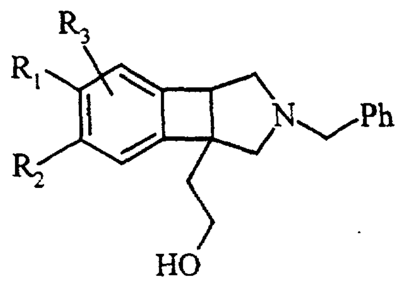

(X)

dans laquelle $R_1$, $R_2$, $R_3$ sont tels que définis précédemment,
composés de formule **(X)** qui sont traités par un composé de formule **(VIII)** :

$R_5$ - OH **(VIII)**

dans laquelle $R_5$ est tel que défini précédemment,
pour conduire aux composés de formule **(I/e)**, à jonction de cycle *cis*, cas particulier des composés de formule (I) :

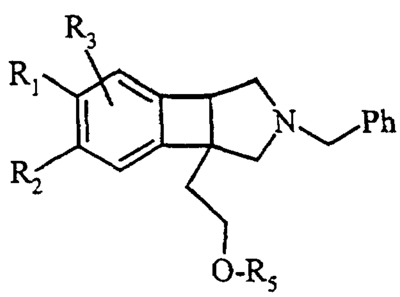

(I/e)

dans laquelle $R_1$, $R_2$, $R_3$ et $R_5$ sont tels que définis précédemment,
composés de formule **(I/e)** qui peuvent être débenzylés par une des méthodes classiquement utilisées en synthèse organique, pour conduire aux composés de formule **(I/f),** à jonction de cycle *cis*, cas particulier des composés de formule (I) :

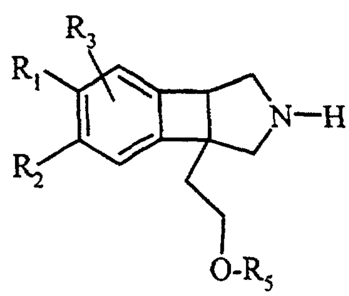

(I/f)

dans laquelle $R_1$, $R_2$, $R_3$ et $R_5$ sont tels que définis précédemment,
composés de formule **(I/f)** qui peuvent être :

* soit transformés par traitement par le formaldéhyde en présence de cyanoborohydrure de sodium en composés de formule **(I/g),** à jonction de cycle *cis*, cas particulier des composés de formule (I) :

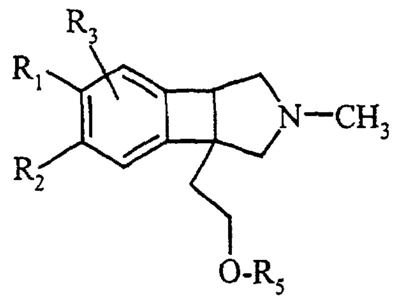

(I/g)

dans laquelle $R_1$, $R_2$, $R_3$ et $R_5$ sont tels que définis précédemment,

* soit mis en présence, selon des conditions classiques de synthèse organique, d'un composé de formule **(IX) :**

$R'_4$ - Z **(IX)**

dans lequel $R'_4$ a les mêmes définitions que $R_4$ à l'exception de hydrogène et benzyle, et Z représente un groupement partant usuellement utilisé en synthèse organique, pour conduire aux composés de formule **(I/h),** à jonction de cycle *cis*, cas particulier des composés de formule (I) :

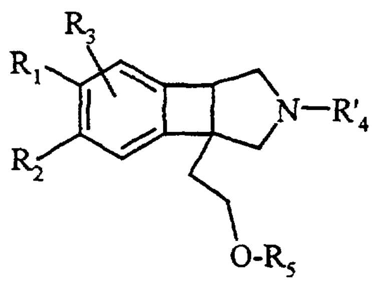

(I/h)

dans laquelle $R_1$, $R_2$, $R_3$, $R'_4$ et $R_5$ sont tels que définis précédemment,

→ soit transformés par une suite de réactions classiques en composés de formule **(XI),** à jonction de cycle *cis*, :

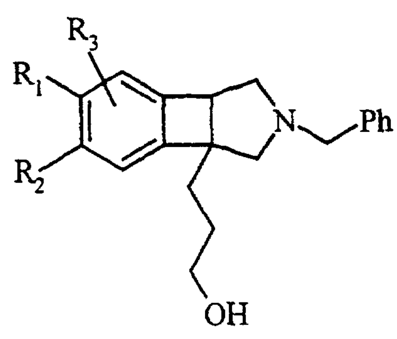

(XI)

dans laquelle $R_1$, $R_2$, $R_3$, sont tels que définis précédemment,
les composés de formule (XI) pouvant également être obtenus par une suite de réactions classiques à partir des composés de formule (X),
composés de formule **(XI)** qui sont traités par un composé de formule **(VIII) :**

$R_5$ - OH **(VIII)**

dans laquelle $R_5$ est tel que défini précédemment,
pour conduire aux composés de formule **(I/i),** à jonction de cycle *cis*, cas particulier des composés de formule (I) :

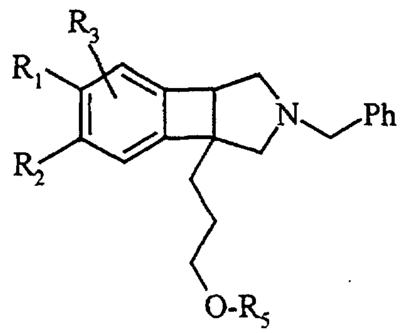

**(I/i)**

dans laquelle $R_1$, $R_2$, $R_3$, et $R_5$ sont tels que définis précédemment,
composés de formule **(I/i)** qui peuvent être débenzylés par une des méthodes classiquement utilisées en synthèse organique pour conduire aux composés de formule **(I/j),** à jonction de cycle *cis*, cas particulier des composés de formule (I) :

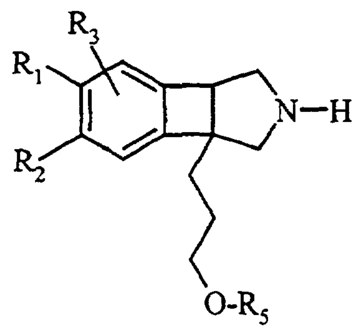

**(I/j)**

dans laquelle $R_1$, $R_2$, $R_3$, et $R_5$ sont tels que définis précédemment,
composés de formule **(I/j)** qui peuvent être :

* soit transformés par traitement par le formaldéhyde en présence de cyanoborohydrure de sodium en composés de formule **(I/k),** à jonction de cycle *cis*, cas particulier des composés de formule (I) :

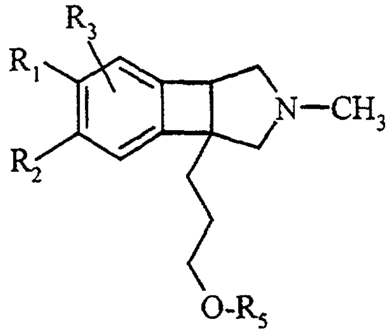

**(I/k)**

dans laquelle $R_1$, $R_2$, $R_3$ et $R_5$ sont tels que définis précédemment,

* soit mis en présence, selon des conditions classiques de synthèse organique, d'un composé de formule

**(IX) :**

$R'_4$ - Z **(IX)**

dans lequel $R'_4$ a les mêmes définitions que $R_4$ à l'exception de hydrogène et benzyle, et Z représente un groupement partant usuellement utilisé en synthèse organique,
pour conduire aux composés de formule **(I/l),** à jonction de cycle *cis*, cas particulier des composés de formule (I) :

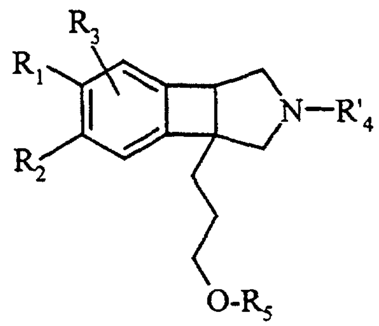

**(I/l)**

dans laquelle $R_1$, $R_2$, $R_3$, $R'_4$ et $R_5$ sont tels que définis précédemment,

les composés **(I/a)** à **(I/l)** formant l'ensemble des composés de l'invention, que l'on purifie, le cas échéant, selon une technique classique de purification, qui peuvent si on le désire, être séparés en leurs différents isomères selon une technique classique de séparation et que l'on transforme, le cas échéant, en leurs sels d'addition à un acide ou à une base pharmaceutiquement acceptable.

**14.** Compositions pharmaceutiques contenant comme principe actif au moins un composé de formule (I) selon l'une quelconque des revendications 1 à 12, seul ou en combinaison avec un ou plusieurs excipients ou véhicules inertes, non toxiques, pharmaceutiquement acceptables.

**15.** Compositions pharmaceutiques selon la revendication 14 contenant au moins un principe actif selon l'une quelconque des revendications 1 à 12, utiles, en temps qu'inhibiteur de la recapture de sérotonine, dans le traitement de la dépression, des attaques de panique, des troubles obsessionnels compulsifs, des phobies, des troubles impulsifs, de l'abus de drogue et de l'anxiété.

## Claims

**1.** Compounds of formula (I), having a *cis* ring junction :

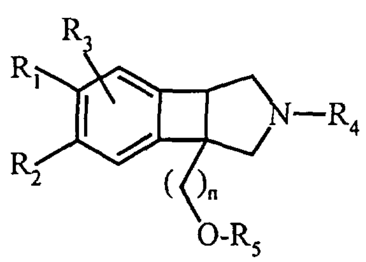

**(I)** ,

wherein :

**$R_1$, $R_2$** and **$R_3$,** which may be the same or different, each independently of the others represents a group selected from a hydrogen atom, a halogen atom, a linear or branched ($C_1$-$C_6$)alkyl group, a linear or branched ($C_2$-$C_6$)alkenyl group, a linear or branched ($C_2$-$C_6$)alkynyl group, a hydroxy group, a linear or branched ($C_1$-$C_6$)

alkoxy group, a cycloalkyl group, a cycloalkyl-($C_1$-$C_6$)alkyl group in which the alkyl moiety is linear or branched, an aryl group, an aryl-($C_1$-$C_6$)alkyl group in which the alkyl moiety is linear or branched, an aryloxy group, an aryl-($C_1$-$C_6$)alkoxy group in which the alkoxy moiety is linear or branched, a linear or branched ($C_1$-$C_6$)trihaloalkyl group, a linear or branched ($C_1$-$C_6$)trihaloalkoxy group, a cyano group, a nitro group and groups of formulae $-OSO_2CH_3$, $-OSO_2CF_3$, $-NR_6R_7$ and $-CO_2R_6$ wherein $R_6$ and $R_7$, which may be the same or different, each independently of the other represents a hydrogen atom, a linear or branched ($C_1$-$C_6$)alkyl group, a linear or branched ($C_2$-$C_6$)alkenyl group, a linear or branched ($C_2$-$C_6$)alkynyl group, a cycloalkyl group, a cycloalkyl-($C_1$-$C_6$)alkyl group in which the alkyl moiety is linear or branched, an aryl group or an aryl-($C_1$-$C_6$) alkyl group in which the alkyl moiety is linear or branched, or two of $R_1$, $R_2$ and $R_3$ in adjacent positions represent, together with the atoms common to the benzene ring to which they are bonded, a saturated or unsaturated ($C_4$-$C_8$)-ring system wherein one or two carbon atoms is/are optionally replaced by one or two hetero atoms, which may be the same or different, selected from oxygen, nitrogen and sulphur, it being understood that in the case where two of $R_1$, $R_2$ and $R_3$ in adjacent positions have the meaning mentioned hereinbefore, the remaining group $R_1$ or $R_2$ or $R_3$ takes one of the above-mentioned individual definitions of those groups,

**$R_4$** represents a group selected from a hydrogen atom, a linear or branched ($C_1$-$C_6$)alkyl group, a linear or branched ($C_2$-$C_6$)alkenyl group, a linear or branched ($C_2$-$C_6$)alkynyl group, a cycloalkyl group, a cycloalkyl-($C_1$-$C_6$)alkyl group in which the alkyl moiety is linear or branched, an aryl group, an aryl-($C_1$-$C_6$)alkyl group in which the alkyl moiety is linear or branched, a heterocycloalkyl group, a heterocycloalkyl-($C_1$-$C_6$)alkyl group in which the alkyl moiety is linear or branched, a heteroaryl group and a heteroaryl-($C_1$-$C_6$)alkyl group in which the alkyl moiety is linear or branched,

**$R_5$** represents an aryl group, an optionally substituted aryl group, a heteroaryl group or an optionally substituted heteroaryl group,

**n** represents an integer from 1 to 3 inclusive,

their isomers and addition salts thereof with a pharmaceutically acceptable acid or base,
a cycloalkyl group being understood to mean a ($C_3$-$C_8$)-ring system, which may be mono- or bi-cyclic, optionally having one or more unsaturated bond(s), the said unsaturated bond(s) not conferring an aromatic character on the ring system, which is optionally substituted by one or more groups, which may be the same or different, selected from a halogen atom and a linear or branched ($C_1$-$C_6$)alkyl group,
a heterocycloalkyl group being understood to mean an optionally substituted cycloalkyl group as defined hereinbefore and containing, in the ring system, one, two or three hetero atoms, which may be the same or different, selected from oxygen, nitrogen and sulphur,
furthermore, an aryl group being understood to mean a phenyl, naphthyl, dihydronaphthyl, tetrahydronaphthyl, indenyl or indanyl group and an optionally substituted aryl group being understood to mean an aryl group, as defined, optionally substituted by one or more groups, which may be the same or different, selected from halogen, hydroxy, linear or branched ($C_1$-$C_6$)alkyl, linear or branched ($C_1$-$C_6$)alkoxy, linear or branched ($C_1$-$C_6$)-alkenyl, cycloalkyl, adamantyl, cycloalkyl-($C_1$-$C_6$)alkyl in which the alkyl moiety is linear or branched, aryl, aryl-($C_1$-$C_6$)alkyl in which the alkyl moiety is linear or branched, aryloxy, aryl-($C_1$-$C_6$)alkoxy in which the alkoxy moiety is linear or branched, linear or branched ($C_1$-$C_6$)trihaloalkyl, linear or branched ($C_1$-$C_6$)trihaloalkoxy, cyano, nitro, oxo, and groups of formulae $-OSO_2CH_3$, $-OSO_2CF_3$, $-NR_6R_7$ and $-CO_2R_6$ wherein $R_6$ and $R_7$ are as defined for formula (I),
a heteroaryl group being likewise understood to mean an aryl group as defined hereinbefore containing one, two or three hetero atoms, which may be the same or different, selected from oxygen, nitrogen and sulphur, and an optionally substituted heteroaryl group being understood to mean a heteroaryl group as defined hereinbefore optionally substituted by one or more groups, which may be the same or different, as defined for substitution of the aryl groups.

2. Compounds of formula (I) according to claim 1, **characterised in that** $R_5$ represents a heteroaryl group, their isomers and addition salts thereof with a pharmaceutically acceptable acid or base.

3. Compounds of formula (I) according to either claim l or claim 2, **characterised in that** $R_5$ represents a methylenedioxyphenyl or ethylenedioxyphenyl group, their isomers and addition salts thereof with a pharmaceutically acceptable acid or base.

4. Compounds of formula (I) according to claim 1, **characterised in that** n is 1, their isomers and addition salts

thereof with a pharmaceutically acceptable acid or base.

**5.** Compounds of formula (I) according to claim 1, **characterised in that** n is 2, their isomers and addition salts thereof with a pharmaceutically acceptable acid or base.

**6.** Compounds of formula (I) according to claim 1, **characterised in that** $R_4$ represents a hydrogen atom, a linear or branched ($C_1$-$C_6$)alkyl group or an aryl-($C_1$-$C_6$)alkyl group in which the alkyl moiety is linear or branched, their isomers and addition salts thereof with a pharmaceutically acceptable acid or base.

**7.** Compound of formula (I) according to claim 1 which is cis-3a-[(3,4-methylenedioxyphenoxy)methyl]-2,3,3a,7b-tetrahydro-1*H*-benzo[3,4]cyclobuta[1,2-c]pyrrole, and its hydrochloride.

**8.** Compound of formula (I) according to claim 1 which is *cis*-2-benzyl-5-methoxy-3a-[(3,4-methylenedioxyphenoxy)-methyl]-2,3,3a,7b-tetrahydro-1*H*-benzo[3,4]cyclobuta-[1,2-c]pyrrole.

**9.** Compound of formula (I) according to claim 1 which is cis-5-methoxy-3a-[(3,4-methylenedioxyphenoxy)-methyl]-2,3,3a,7b-tetrahydro-1*H*-benzo[3,4]cyclobuta[1,2-c]-pyrrole, and its hydrochloride.

**10.** Compound of formula (I) according to claim 1 which is *cis*-5-methoxy-2-methyl-3a-[(3,4-methylenedioxyphenoxy)-methyl]-2,3,3a,7b-tetrahydro-1*H*-benzo[3,4]cyclobuta[1,2-c]pyrrole.

**11.** Compound of formula (I) according to claim 1 which is *cis*-6-trifluoromethyl-3a-[(3,4-methylenedioxyphenoxy)-methyl]-2,3,3a,7b-tetrahydro-1*H*-benzo[3,4]cyclobuta[1,2-c]-pyrrole, and its hydrochloride.

**12.** Compound of formula (I) according to claim 1 which is *cis*-6-fluoro-3a-[(3,4-methylenedioxyphenoxy)-methyl]-2,3,3a,7b-tetrahydro-1*H*-benzo[3,4]cyclobuta[1,2-c]pyrrole, and its hydrochloride.

**13.** Process for the preparation of compounds of formula (I) according to claim 1, **characterised in that** there is used as starting material a compound of formula (II) :

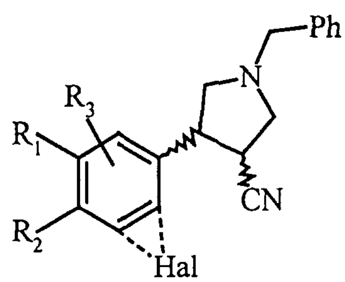

**(II)**,

wherein $R_1$, $R_2$ and $R_3$ are as defined for formula **(I)** and Hal represents a halogen atom, the said halogen atom being attached to either of the carbon atoms of the benzene ring,
which compounds of formula **(II)** are subjected to the action of sodium amide in liquid ammonia to yield the compounds of formula **(III),** having a *cis* ring junction,

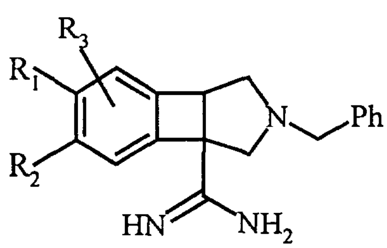

**(III)**,

wherein $R_1$, $R_2$ and $R_3$ are as defined for formula **(I),**
which compounds of formula **(III)** are treated with a mineral base, in an aqueous alcoholic medium, to yield the

compounds of formula **(IV),** having a *cis* ring junction,

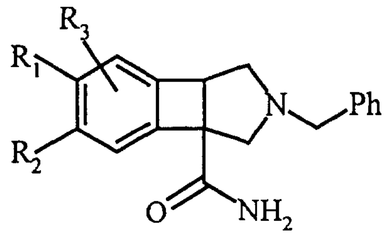

**(IV)** ,

wherein $R_1$, $R_2$ and $R_3$ are as defined for formula **(I),**
which compounds of formula **(IV)** are converted, by means of an acetal of N,N-dimethylformamide of formula **(V)**

$$(CH_3)_2N\ CH(OG)_2 \qquad \textbf{(V)},$$

wherein G represents a linear or branched ($C_1$-$C_6$)alkyl, a benzyl or a cyclohexyl group,
into an ester of formula **(VI),** having a *cis* ring junction,

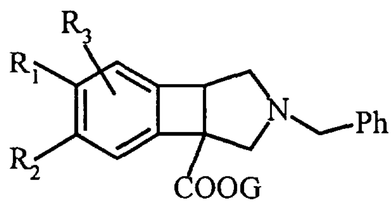

**(VI)** ,

wherein $R_1$, $R_2$, $R_3$ and G are as defined hereinbefore,
which compounds of formula **(VI)** are reduced, under conventional conditions of organic synthesis, to an alcohol of formula **(VII),** having a *cis* ring junction,

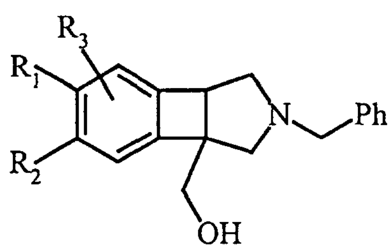

**(VII)** ,

wherein $R_1$, $R_2$ and $R_3$ are as defined hereinbefore,
which compounds of formula **(VII)** are :

→ either treated with a compound of formula **(VIII)**

$$R_5 - OH \qquad \textbf{(VIII)},$$

wherein $R_5$ is as defined for formula **(I),**
to yield the compounds of formula **(I/a),** having a *cis* ring junction, a particular case of the compounds of formula (I) :

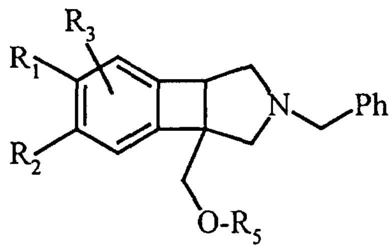 **(I/a)**,

wherein $R_1$, $R_2$, $R_3$ and $R_5$ are as defined hereinbefore,
which compounds of formula **(I/a)** may be debenzylated by one of the methods conventionally used in organic synthesis, to yield the compounds of formula **(I/b),** having a *cis* ring junction, a particular case of the compounds of formula (I):

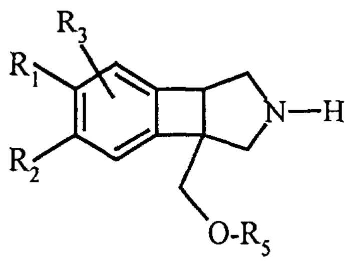 **(I/b)**,

wherein $R_1$, $R_2$, $R_3$ and $R_5$ are as defined hereinbefore,
which compounds of formula **(I/b)** may be :

* either converted, by treating with formaldehyde in the presence of sodium cyanoborohydride, into compounds of formula **(I/c)**, having a *cis* ring junction, a particular case of the compounds of formula (I) :

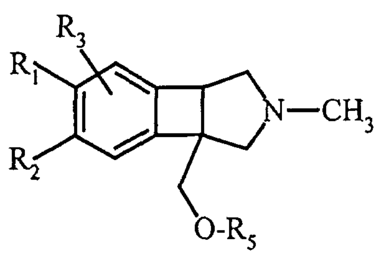 **(I/c)**,

wherein $R_1$, $R_2$, $R_3$ and $R_5$ are as defined hereinbefore,

* or treated, under conventional conditions of organic synthesis, with a compound of formula **(IX) :**

$$R'_4 - Z \qquad \textbf{(IX)},$$

wherein $R'_4$ has the same meanings as $R_4$ except hydrogen and benzyl, and Z represents a leaving group customarily used in organic synthesis,
to yield the compounds of formula **(I/d),** having a *cis* ring junction, a particular case of the compounds of formula (I) :

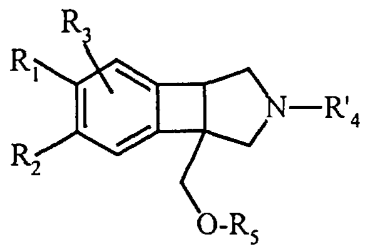

**(I/d)** ,

wherein $R_1$, $R_2$, $R_3$, $R'_4$ and $R_5$ are as defined hereinbefore,

→ or converted, by a succession of conventional reactions, into compounds of formula **(X),** having a *cis* ring junction,

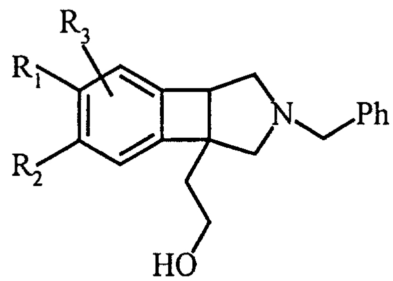

**(X)** ,

wherein $R_1$, $R_2$ and $R_3$ are as defined hereinbefore,
which compounds of formula **(X)** are treated with a compound of formula **(VIII) :**

$R_5$ - OH **(VIII),**

wherein $R_5$ is as defined hereinbefore,
to yield the compounds of formula **(I/e),** having a *cis* ring junction, a particular case of the compounds of formula (I) :

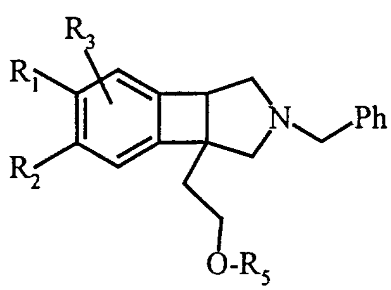

**(I/e)** ,

wherein $R_1$, $R_2$, $R_3$ and $R_5$ are as defined hereinbefore,
which compounds of formula **(I/e)** may be debenzylated by one of the methods conventionally used in organic synthesis, to yield the compounds of formula **(I/f),** having a *cis* ring junction, a particular case of the compounds of formula (I) :

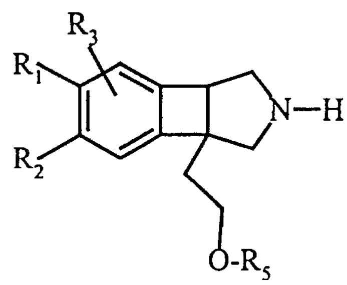

**(I/f)**,

wherein $R_1$, $R_2$, $R_3$ and $R_5$ are as defined hereinbefore, which compounds of formula **(I/f)** may be :

* either converted, by treating with formaldehyde in the presence of sodium cyanoborohydride, into compounds of formula **(I/g),** having a *cis* ring junction, a particular case of the compounds of formula (I) :

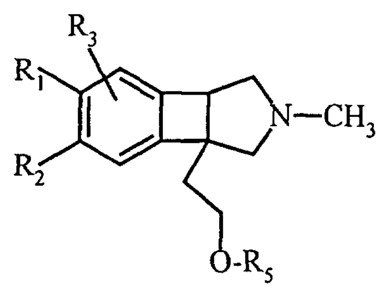

**(I/g)**,

wherein $R_1$, $R_2$, $R_3$ and $R_5$ are as defined hereinbefore,

* or treated, under conventional conditions of organic synthesis, with a compound of formula **(IX) :**

$$R'_4 - Z \qquad \textbf{(IX)},$$

wherein $R'_4$ has the same meanings as $R_4$ except hydrogen and benzyl, and Z represents a leaving group customarily used in organic synthesis,
to yield the compounds of formula **(I/h),** having a *cis* ring junction, a particular case of the compounds of formula (I) :

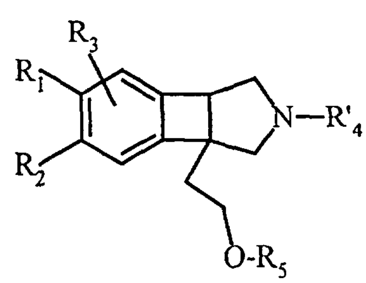

**(I/h)**,

wherein $R_1$, $R_2$, $R_3$, $R'_4$ and $R_5$ are as defined hereinbefore,

→ or converted, by a succession of conventional reactions, into compounds of formula **(XI),** having a *cis* ring junction :

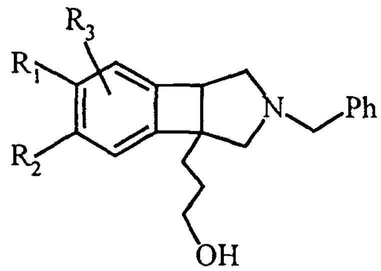
**(XI)**,

wherein $R_1$, $R_2$ and $R_3$ are as defined hereinbefore,
it also being possible for the compounds of formula (XI) to be obtained by a succession of conventional reactions starting from compounds of formula (X),
which compounds of formula **(XI)** are treated with a compound of formula **(VIII) :**

$R_5$ - OH **(VIII),**

wherein $R_5$ is as defined hereinbefore,
to yield the compounds of formula **(I/i),** having a *cis* ring junction, a particular case of the compounds of formula (I) :

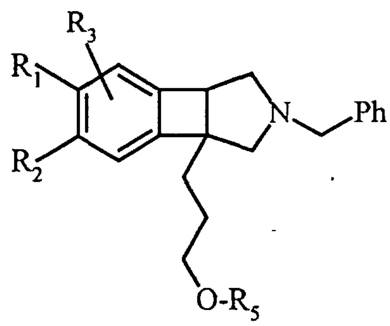
**(I/i)**,

wherein $R_1$, $R_2$, $R_3$ and $R_5$ are as defined hereinbefore,
which compounds of formula **(I/i)** may be debenzylated by one of the methods conventionally used in organic synthesis to yield the compounds of formula **(I/j),** having a *cis* ring junction, a particular case of the compounds of formula (I) :

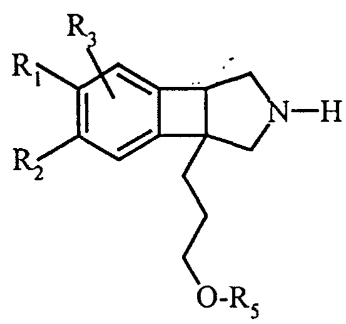
**(I/j)**,

wherein $R_1$, $R_2$, $R_3$ and $R_5$ are as defined hereinbefore,
which compounds of formula **(I/j)** may be :

* either converted, by treating with formaldehyde in the presence of sodium cyanoborohydride, into compounds of formula **(I/k),** having a *cis* ring junction, a particular case of the compounds of formula (I) :

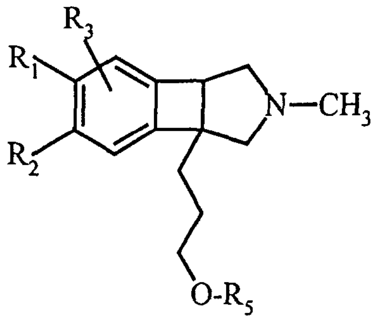

**(I/k)** ,

wherein $R_1$, $R_2$, $R_3$ and $R_5$ are as defined hereinbefore,

* or treated, under conventional conditions of organic synthesis, with a compound of formula **(IX) :**

$$R'_4 - Z \qquad \textbf{(IX)},$$

wherein $R'_4$ has the same meanings as $R_4$ except hydrogen and benzyl, and Z represents a leaving group customarily used in organic synthesis,
to yield the compounds of formula **(I/l),** having a *cis* ring junction, a particular case of the compounds of formula (I):

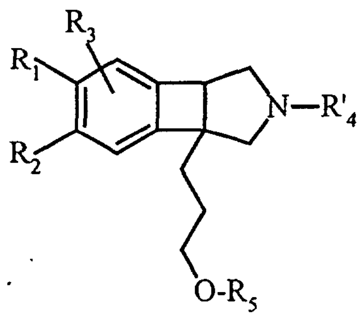

**(I/l)**,

wherein $R_1$, $R_2$, $R_3$, $R'_4$ and $R_5$ are as defined hereinbefore,

which compounds **(I/a)** to **(I/l)** constitute the totality of the compounds of the invention, which are purified, if necessary, according to a conventional purification technique, which may be separated, if desired, into their various isomers according to a conventional separation technique, and which are converted, where appropriate, into addition salts thereof with a pharmaceutically acceptable acid or base.

**14.** Pharmaceutical compositions comprising as active ingredient at least one compound of formula (I) according to any one of claims 1 to 12, alone or in combination with one or more inert, non-toxic, pharmaceutically acceptable excipients or carriers.

**15.** Pharmaceutical compositions according to claim 14 comprising at least one active ingredient according to any one of claims 1 to 12 for use, as an inhibitor of serotonin reuptake, in the treatment of depression, panic attacks, obsessive-compulsive disorders, phobias, impulsive disorders, drug abuse and anxiety.

## Patentansprüche

**1.** Verbindungen der Formel (I) mit *cis*-Ringbindung:

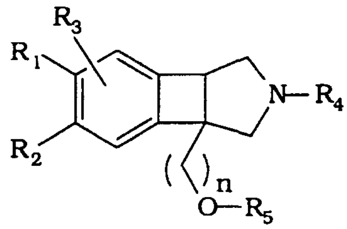

(I)

in der:

$R_1$, $R_2$ und $R_3$, die gleichartig oder verschieden sind, unabhängig voneinander eine Gruppe ausgewählt aus Wasserstoffatomen, Halogenatomen, geradkettigen oder verzweigten $(C_1-C_6)$-Alkylgruppen, geradkettigen oder verzweigten $(C_2-C_6)$-Alkenylgruppen, geradkettigen oder verzweigten $(C_2-C_6)$-Alkinylgruppen, Hydroxygruppen, geradkettigen oder verzweigten $(C_1-C_6)$-Alkoxygruppen, Cycloalkylgruppen, geradkettigen oder verzweigten Cycloalkyl-$(C_1-C_6)$-alkylgruppen, Arylgruppen, geradkettigen oder verzweigten Aryl-$(C_1-C_6)$-alkylgruppen, Aryloxygruppen, geradkettigen oder verzweigten Aryl-$(C_1-C_6)$-alkoxygruppen, geradkettigen oder verzweigten $(C_1-C_6)$-Trihalogenalkylgruppen, geradkettigen oder verzweigten $(C_1-C_6)$-Trihalogenoxygruppen, Cyanogruppen, Nitrogruppen, Gruppen der Formeln $-OSO_2CH_3$, $-OSO_2CF_3$, $NR_6R_7$, $CO_2R_6$, worin $R_6$ und $R_7$, die gleichartig oder verschieden sind, unabhängig voneinander jeweils Wasserstoffatome, geradkettige oder verzweigte $(C_1-C_6)$-Alkylgruppen, geradkettige oder verzweigte $(C_2-C_6)$-Alkenylgruppen, geradkettigen oder verzweigte $(C_2-C_6)$-Alkinylgruppen, Cycloalkylgruppen, geradkettige oder verzweigte Cycloalkyl-$(C_1-C_6)$-alkylgruppen, Arylgruppen, geradkettige oder verzweigte Aryl-$(C_1-C_6)$-alkylgruppen darstellen, bedeuten, oder jeweils zwei von $R_1$, $R_2$ und $R_3$, in benachbarten Stellungen zusammen mit den gemeinsamen Atomen des Benzolrings, an den sie gebunden sind, ein gesättigtes oder ungesättigtes cyclisches $(C_4-C_8)$-System bilden, bei dem gegebenenfalls ein oder zwei Kohlenstoffatome durch ein oder zwei gleichartige oder verschiedene Heteroatome, ausgewählt aus Sauerstoff, Stickstoff und Schwefel, ersetzt sind, mit der Maßgabe, daß, wenn zwei der Gruppen $R_1$, $R_2$ und $R_3$ in benachbarten Stellungen vorliegen und die oben angegebene Definition erfüllen, die verbleibende Gruppe $R_1$ oder $R_2$ oder $R_3$ einer der oben definierten individuellen Definitionen dieser Gruppen entspricht,

$R_4$ eine Gruppe ausgewählt aus Wasserstoff, geradkettigen oder verzweigten $(C_1-C_6)$-Alkylgruppen, geradkettigen oder verzweigten $(C_2-C_6)$-Alkenylgruppen, geradkettigen oder verzweigten $(C_2-C_6)$-Alkinylgruppen, Cycloalkylgruppen, geradkettigen oder verzweigten Cycloalkyl-$(C_1-C_6)$-alkylgruppen, Arylgruppen, geradkettigen oder verzweigten Aryl-$(C_1-C_6)$-alkylgruppen, Heterocycloalkylgruppen, geradkettigen oder verzweigten Heterocycloalkyl-$(C_1-C_6)$-alkylgruppen, Heteroarylgruppen und geradkettigen oder verzweigten Heteroaryl-$(C_1-C_6)$-alkylgruppen bedeutet,

$R_5$ eine Arylgruppe, eine gegebenenfalls substituierte Arylgruppe, eine Heteroarylgruppe oder eine gegebenenfalls substituierte Heteroarylgruppe darstellt,

**n** eine ganze Zahl mit einem Wert zwischen 1 und 3 einschließlich bedeutet,

deren Isomere sowie deren Additionssalze mit einer pharmazeutisch annehmbaren Säure oder Base,
mit der Maßgabe, daß unter einer Cycloalkylgruppe ein mono- oder bicyclisches cyclisches $(C_3-C_8)$-System zu verstehen ist, welches gegebenenfalls eine oder mehrere Unsättigungen aufweist, welche Unsättigungen dem cyclischen System jedoch keinen aromatischen Charakter verleihen, und welches gegebenenfalls durch eine oder mehrere, gleichartige oder verschiedene Gruppen ausgewählt aus Halogenatomen und geradkettigen oder verzweigten $(C_1-C_6)$-Alkylgruppen substituiert sein kann,
mit der Maßgabe, daß unter einer Heterocycloalkylgruppe eine gegebenenfalls substituierte Cycloalkylgruppe zu verstehen ist, wie sie oben definiert worden ist, und die im Bereich des cyclischen Systems ein, zwei oder drei gleichartige oder verschiedene Heteroatome ausgewählt aus Sauerstoff, Stickstoff und Schwefel aufweist,
wobei es sich versteht, daß unter einer Arylgruppe eine Phenyl-, Naphthyl-, Dihydronaphthyl-, Tetrahydronaphthyl-, Indenyl- oder Indanyl-gruppe zu verstehen ist, und unter einer gegebenenfalls substituierten Arylgruppe eine Arylgruppe der definierten Art zu verstehen ist, die gegebenenfalls durch eine oder mehrere gleichartige oder verschiedene Gruppen substituiert ist ausgewählt aus Halogen, Hydroxy, geradkettigem oder verzweigtem $(C_1-C_6)$ -Alkyl, geradkettigem oder verzweigtem $(C_1-C_6)$-Alkoxy, geradkettigem oder verzweigtem $(C_1-C_6)$-Alkenyl, Cycloalkyl, Adamantyl, geradkettigem oder verzweigtem Cycloalkyl-$(C_1-C_6)$-alkyl, Aryl, geradkettigem oder verzweigtem Aryl-$(C_1-C_6)$-alkyl, Aryloxy, geradkettigem oder verzweigtem Aryl-$(C_1-C_6)$-alkoxy, geradkettigem oder verzweigtem $(C_1-C_6)$-Trihalogenalkyl, geradkettigem oder verzweigtem $(C_1-C_6)$-Trihalogenalkoxy, Cyano, Nitro, Oxo, Gruppen

der Formeln $-OSO_2CH_3$, $-OSO_2CF_3$, $NR_6R_7$ oder $CO_2R_6$, worin $R_6$ und $R_7$ die bezüglich der Formel (I) angegebenen Bedeutungen besitzen,
wobei es sich weiterhin versteht, daß unter einer Heteroarylgruppe eine Arylgruppe der oben definierten Art zu verstehen ist, die ein, zwei oder drei gleichartige oder verschiedene Heteroatome ausgewählt aus Sauerstoff, Stickstoff und Schwefel enthält, und daß unter einer gegebenenfalls substituierten Heteroarylgruppe eine Heteroarylgruppe der oben definierten Art zu verstehen ist, die gegebenenfalls durch eine oder mehrere gleichartige oder verschiedene Gruppen, wie sie oben für die Substituenten der Arylgruppen definiert worden sind, substituiert ist.

**2.** Verbindungen der Formel (I) nach Anspruch 1, **dadurch gekennzeichnet, daß** $R_5$ eine Heteroarylgruppe darstellt, deren Isomere sowie deren Additionssalze mit einer pharmazeutisch annehmbaren Säure oder Base.

**3.** Verbindungen der Formel (I) nach einem der Ansprüche 1 oder 2, **dadurch gekennzeichnet, daß** $R_5$ eine Methylendioxyphenylgruppe oder eine Ethylendioxyphenylgruppe darstellt, deren Isomeren sowie deren Additionssalze mit einer pharmazeutisch annehmbaren Säure oder Base.

**4.** Verbindungen der Formel (I) nach Anspruch 1, **dadurch gekennzeichnet, daß** n den Wert 1 besitzt, deren Isomere sowie deren Additionssalze mit einer pharmazeutisch annehmbaren Säure oder Base.

**5.** Verbindungen der Formel (I) nach Anspruch 1, **dadurch gekennzeichnet, daß** n den Wert 2 besitzt, deren Isomere sowie deren Additionssalze mit einer pharmazeutisch annehmbaren Säure oder Base.

**6.** Verbindungen der Formel (I) nach Anspruch 1, **dadurch gekennzeichnet, daß** $R_4$ ein Wasserstoffatom, eine geradkettige oder verzweigte $(C_1-C_6)$-Alkylgruppe oder eine geradkettige oder verzweigte Aryl-$(C_1-C_6)$-alkylgruppe bedeutet, deren Isomere sowie deren Additionssalze mit einer pharmazeutisch annehmbaren Säure oder Base.

**7.** Verbindung der Formel (I) nach Anspruch 1, nämlich *cis*-3a-[(3,4-Methylendioxyphenyloxy)-methyl]-2,3,3a,7b-tetrahydro-1*H*-benzo[3,4]cyclobuta[1,2-c]pyrrol und dessen Hydrochlorid.

**8.** Verbindung der Formel (I) nach Anspruch 1, nämlich *cis*-2-Benzyl-5-methoxy-3a-[(3,4-methylendioxyphenyloxy)-methyl]-2,3,3a,7b-tetrahydro-1*H*-benzo-[3,4]cyclobuta[1,2-c]pyrrol.

**9.** Verbindung der Formel (I) nach Anspruch 1, nämlich *cis*-5-Methoxy-3a-[(3,4-methylendioxyphenyloxy)-methyl]-2,3,3a,7b-tetrahydro-1*H*-benzo[3,4]cyclobuta[1,2-c]pyrrol und dessen Hydrochlorid.

**10.** Verbindung der Formel (I) nach Anspruch 1, nämlich *cis*-5-Methoxy-2-methyl-3a-[(3,4-methylendioxyphenyloxy)-methyll-2,3,3a,7b-tetrahydro-1*H*-benzo-[3,4]cyclobuta[1,2-c]pyrrol.

**11.** Verbindung der Formel (I) nach Anspruch 1, nämlich *cis*-6-Trifluormethyl-3a-[(3,4-methylendioxyphenyloxy)-methyl]-2,3,3a,7b-tetrahydro-1*H*-benzo[3,4]cyclobuta[1,2-c]pyrrol und dessen Hydrochlorid.

**12.** Verbindung der Formel (I) nach Anspruch 1, nämlich *cis*-6-Fluor-3a-[(3,4-methylendioxyphenyloxy)-methyl]-2,3,3a,7b-tetrahydro-1*H*-benzo[3,4]cyclobuta-[1,2-c]pyrrol und dessen Hydrochlorid.

**13.** Verfahren zur Herstellung der Verbindungen der Formel (I) nach Anspruch 1, **dadurch gekennzeichnet, daß** man als Ausgangsprodukt eine Verbindung der Formel (II) verwendet:

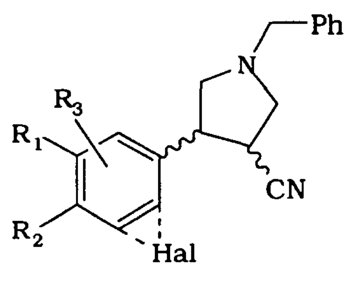

(II)

in der $R_1$, $R_2$ und $R_3$ die bezüglich der Formel **(I)** angegebenen Bedeutungen besitzen und Hal ein Halogenatom bedeutet, welches Halogenatom an dem einen oder dem anderen Kohlenstoffatom des Benzolrings gebunden ist, welche Verbindungen der Formel (**II**) man der Einwirkung von Natriumamid in flüssigem Ammoniak unterwirft zur Bildung der Verbindungen der Formel (**III**) mit einer *cis*-Ringbindung

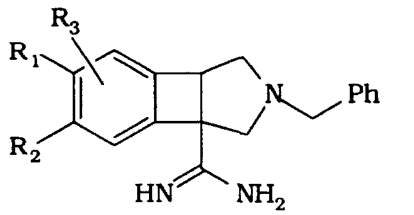

**(III)**

in der $R_1$, $R_2$ und $R_3$ die bezüglich der Formel **(I)** angegebenen Bedeutungen besitzen,
welche Verbindungen der Formel **(III)** man mit einer anorganischen Base in wäßrigem alkoholischem Medium behandelt zur Bildung der Verbindungen der Formel **(IV)** mit *cis*-Ringbindung

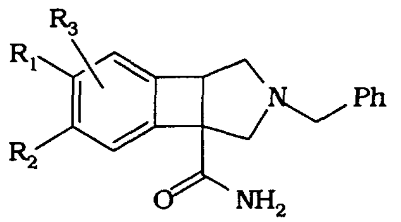

**(IV)**

in der $R_1$, $R_2$ und $R_3$ die bezüglich der Formel **(I)** angegebenen Bedeutungen besitzen,
welche Verbindungen der Formel **(IV)** man mit einem N,N-Dimethylformamid-acetal der Formel **(V):**

$$(CH_3)_2N\ CH(OG)_2 \qquad \textbf{(V)}$$

in der G eine geradkettige oder verzweigte ($C_1$-$C_6$)-Alkylgruppe, eine Benzylgruppe oder eine Cyclohexylgruppe darstellt,
in einen Ester der Formel **(VI)** mit *cis*-Ringbindung umwandelt

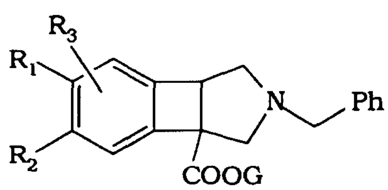

**(VI)**

in der $R_1$, $R_2$, $R_3$ und G die oben angegebenen Bedeutungen besitzen,
welche Verbindungen der Formel **(VI)** man unter den klassischen Bedingungen oder organischen Synthese zu einem Alkohol der Formel **(VII)** mit *cis*-Ringbindung reduziert

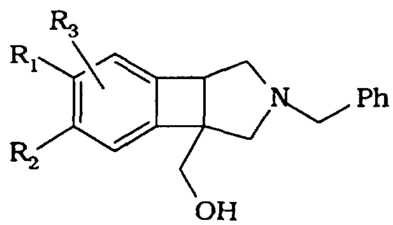
(VII)

in der $R_1$, $R_2$ und $R_3$ die oben angegebenen Bedeutungen besitzen, welche Verbindungen der Formel **(VII):**

→ entweder mit einer Verbindung der Formel **(VIII):**

$$R_5 - OH \qquad \textbf{(VIII)}$$

in der $R_5$ die bezüglich der Formel (**I**) angegebenen Bedeutungen besitzt, behandelt werden zur Bildung der Verbindungen der Formel **(I/a)** mit *cis*-Ringbindung, einem Sonderfall der Verbindungen der Formel (I):

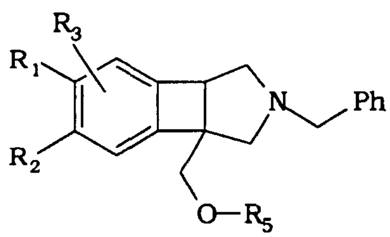
(I/a)

in der $R_1$, $R_2$, $R_3$ und $R_5$ die oben angegebenen Bedeutungen besitzen, welche Verbindungen der Formel **(I/a)** mit Hilfe einer in klassischer Weise in der organischen Synthese angewandten Methoden debenzyliert werden können zur Bildung der Verbindungen der Formel **(I/b),** mit *cis*-Ringbindung, einem Sonderfall der Verbindungen der Formel (I):

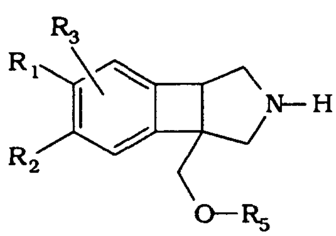
(I/b)

in der $R_1$, $R_2$, $R_3$ und $R_5$ die oben angegebenen Bedeutungen besitzen, welche Verbindungen der Formel **(I/b):**

* entweder durch Behandeln mit Formaldehyd in Gegenwart von Natriumcyanborhydrid zu den Verbindungen der Formel **(I/c)** mit *cis*-Ringbindung umgewandelt werden können, einem Sonderfall der Verbindungen der Formel **(I):**

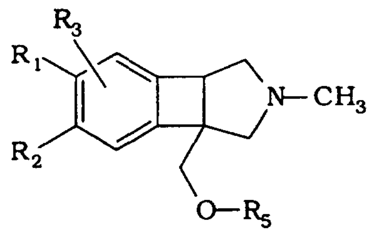

**(I/c)**

in der $R_1$, $R_2$, $R_3$ und $R_5$ die oben angegebenen Bedeutungen besitzen,

* oder unter Anwendung klassischer Bedingungen der organischen Synthese mit einer Verbindung der Formel **(IX):**

$R'_4$ - Z **(IX)**

in der $R'_4$ die oben für $R_4$ angegebenen Bedeutungen besitzt mit Ausnahme von Wasserstoff und Benzyl, und

Z eine üblicherweise in der organischen Synthese verwendete austretende Gruppe darstellt,

behandelt wird zur Bildung der Verbindungen der Formel **(I/d)** mit *cis*-Ringbindung, einem Sonderfall der Verbindungen der Formel (I):

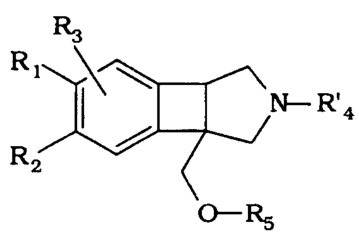

**(I/d)**

in der $R_1$, $R_2$, $R_3$, $R'_4$ und $R_5$ die oben angegebenen Bedeutungen besitzen,

→ oder durch eine Folge von klassischen Reaktionen in die Verbindungen der Formel **(X)** mit *cis*-Ringbindung umgewandelt werden können:

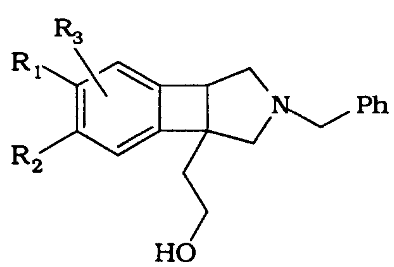

**(X)**

in der $R_1$, $R_2$ und $R_3$ die oben angegebenen Bedeutungen besitzen,
welche Verbindungen der Formel (X) mit einer Verbindung der Formel **(VIII):**

$R_5$ - OH **(VIII)**

in der $R_5$ die oben angegebenen Bedeutungen besitzt,
behandelt werden zur Bildung der Verbindungen der Formel **(I/e)** mit *cis*-Ringbindung, einem Sonderfall der Verbindungen der Formel (I):

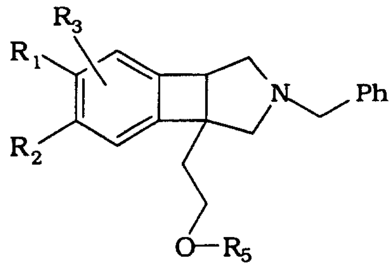

**(I/e)**

in der $R_1$, $R_2$, $R_3$ und $R_5$ die oben angegebenen Bedeutungen besitzen,
welche Verbindungen der Formel **(I/e)** mit Hilfe einer klassischerweise in der organischen Synthese angewandten Methode debenzyliert werden können zur Bildung der Verbindungen der Formel **(I/f)** mit *cis*-Ringbindung, einem Sonderfall der Verbindungen der Formel (I):

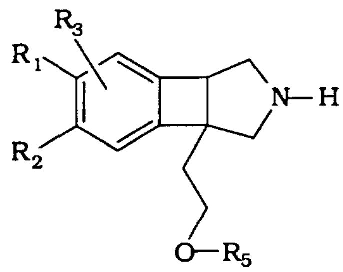

**(I/f)**

in der $R_1$, $R_2$, $R_3$ und $R_5$ die oben angegebenen Bedeutungen besitzen, welche Verbindungen der Formel **(I/f):**

* entweder durch Behandlung mit Formaldehyd in Gegenwart von Natriumcyanborhydrid in die Verbindungen der Formel **(I/g)** mit *cis*-Ringbindung umgewandelt werden können, einem Sonderfall der Verbindungen der Formel (I):

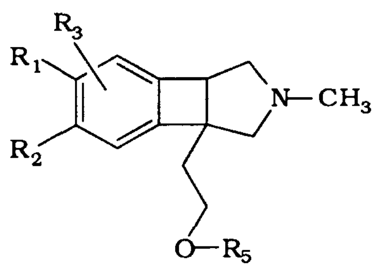

**(I/g)**

in der $R_1$, $R_2$, $R_3$ und $R_5$ die oben angegebenen Bedeutungen besitzen,

* oder unter Anwendung klassischer Bedingungen der organischen Synthese mit einer Verbindung der Formel **(IX):**

$$R'_4 - Z \qquad \textbf{(IX)}$$

in der $R'_4$ die gleichen Bedeutungen besitzt wie $R_4$ mit der Ausnahme von Wasserstoff und Benzyl, und Z eine üblicherweise in der organischen Synthese verwendete austretende Gruppe darstellt, behandelt werden können zur Bildung der Verbindungen der Formel **(I/h),** mit *cis*-Ringbindung, einem Sonderfall der Verbindungen der Formel (I):

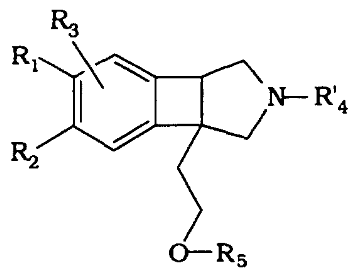

(I/h)

in der $R_1$, $R_2$, $R_3$, $R'_4$ und $R_5$ die oben angegebenen Bedeutungen besitzen,

→ oder mit einer Folge von klassischen Reaktionen in die Verbindungen der Formel **(XI)** mit *cis*-Ringbindung umgewandelt werden können:

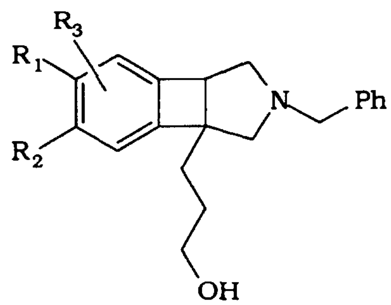

(XI)

in der $R_1$, $R_2$ und $R_3$ die oben angegebenen Bedeutungen besitzen,
welche Verbindungen der Formel (XI) ebenfalls erhalten werden können durch eine Folge von klassischen Reaktionen ausgehend von den Verbindungen der Formel (X),
welche Verbindungen der Formel **(XI)** mit einer Verbindung der Formel **(VIII):**

$R_5$ - OH **(VIII)**

in der $R_5$ die oben angegebenen Bedeutungen besitzt,
behandelt werden zur Bildung der Verbindungen der Formel **(I/i)** mit cts-Ringbindung, einem Sonderfall der Verbindungen der Formel (I):

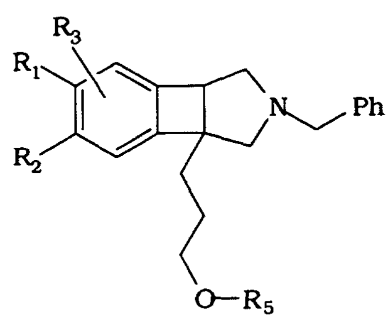

(I/i)

in der $R_1$, $R_2$, $R_3$ und $R_5$ die oben angegebenen Bedeutungen besitzen,
welche Verbindungen der Formel **(I/i)** mit Hilfe klassischerweise in der organischen Synthese verwendeten Methoden debenzyliert werden können zur Bildung der Verbindungen der Formel **(I/j)** mit *cis*-Ringbindung, einem Sonderfall der Verbindungen der Formel (I):

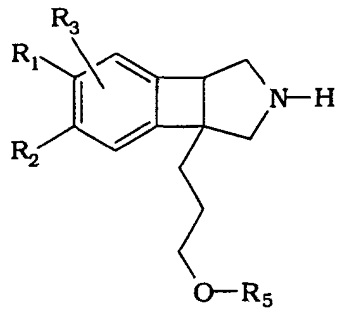

**(I/j)**

in der $R_1$, $R_2$, $R_3$ und $R_5$ die oben angegebenen Bedeutungen besitzen, welche Verbindungen der Formel **(I/j):**

* entweder durch Behandeln mit Formaldehyd in Gegenwart von Natriumcyanborhydrid in die Verbindungen der Formel **(I/k)** mit *cis*-Ringbindung umgewandelt werden können, einem Sonderfall der Verbindungen der Formel (I):

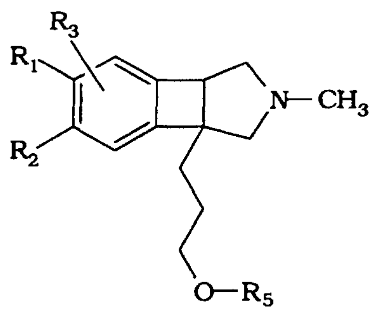

**(I/k)**

in der $R_1$, $R_2$, $R_3$ und $R_5$ die oben angegebenen Bedeutungen besitzen,

* oder unter Anwendung klassischer Bedingungen der organischen Synthese mit einer Verbindung der Formel **(IX):**

$$R'_4 - Z \qquad \textbf{(IX)}$$

in der $R'_4$ die gleichen Bedeutungen besitzt wie $R_4$ mit Ausnahme von Wasserstoff und Benzyl, und Z eine üblicherweise in der organischen Synthese verwendete austretende Gruppe darstellt, behandelt werden können zur Bildung der Verbindungen der Formel **(I/1)** mit *cis*-Ringbindung, einem Sonderfall der Verbindungen der Formel (I):

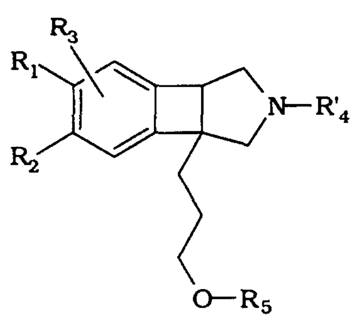

**(I/1)**

in der $R_1$, $R_2$, $R_3$, $R'_4$ und $R_5$ die oben angegebenen Bedeutungen besitzen,

welche Verbindungen der Formeln **(I/a)** bis **(I/kl)** die Gesamtheit der Verbindungen der Erfindung bilden, welche man gegebenenfalls mit Hilfe einer klassischen Reinigungsmethode reinigt, welche gewünschtenfalls mit

Hilfe einer klassischen Trennmethode in ihre verschiedenen Isomeren aufgetrennt werden können und welche gegebenenfalls in ihre Additionssalze mit einer pharmazeutisch annehmbaren Säure oder Base umgewandelt werden können.

**14.** Pharmazeutische Zubereitungen enthaltend als Wirkstoff mindestens eine Verbindung der Formel (I) nach einem der Ansprüche 1 bis 12 allein oder in Kombination mit einem oder mehreren inerten nichttoxischen, pharmazeutisch annehmbaren Trägermaterialien oder Bindemitteln.

**15.** Pharmazeutische Zubereitungen nach Anspruch 14 enthaltend mindestens einen Wirkstoff nach einem der Ansprüche 1 bis 12 als Inhibitoren für das Wiedereinfangen von Serotonin bei der Behandlung von Depressionen, Panikanfällen, kompulsiven Besessenheitsstörungen, Phobien, impulsiven Störungen, dem Drogenmißbrauch und der Angst.